# EUROPEAN PATENT SPECIFICATION

(11) **EP 1 497 656 B1**
(45) Date of publication and mention of the grant of the patent: **29.11.2006**
(21) Application number: 03717443.0
(22) Date of filing: 10.04.2003
(51) Int. Cl.: G01N 33/574

(54) **DIAGNOSIS OF CARCINOMA USING RAIG1 POLYPEPTIDES**
DIAGNOSE VON KARZINOMEN MIT HILFE DES RAIG1-POLYPEPTIDS
DIAGNOSTIC DES CARCINOMES UTILISANT LE POLYPEPTIDE RAIG1

(30) Priority: 11.04.2002 GB 0208331; 17.09.2002 GB 0221538
(43) Date of publication of application: 19.01.2005
(73) Proprietor: UCB, S.A., 1070 Bruxelles (BE)
(72) Inventor: TERRETT, J. A., Oxford GlycoSciences (UK) Ltd, Abingdon, Oxfordshire OX14 4RY (GB)
(74) Representative: Thompson, John
(86) International application number: PCT/GB2003/001587
(87) International publication number: WO 2003/087832

(56) References cited:
- CHENG AND R LOTAN Y: "Molecular cloning and characterization of a novel retinoic acid-inducible gene that encodes a putative G protein-coupled receptor" JOURNAL OF BIOLOGICAL CHEMISTRY, AMERICAN SOCIETY OF BIOLOGICAL CHEMISTS, BALTIMORE, MD, US, vol. 273, no. 52, 25 December 1998 (1998-12-25), pages 35008-35015, XP002108015 ISSN: 0021-9258 cited in the application
- BRAUNER-OSBORNE H ET AL: "Sequence and Expression Pattern of a Novel Human Orphan G-Protein-Coupled Receptor, GPRC5B, a Family C Receptor with a Short Amino-Terminal Domain" GENOMICS, ACADEMIC PRESS, SAN DIEGO, US, vol. 65, no. 2, 15 April 2000 (2000-04-15), pages 121-128, XP004439377 ISSN: 0888-7543 cited in the application
- GYGI S ET AL: "Correlation between Protein and mRNA Abundance in Yeast" MOLECULAR AND CELLULAR BIOLOGY, vol. 19, no. 3, March 1999 (1999-03), pages 1720-1730, XP002923559

### SEQUENCE LISTING

<110> Oxford GlycoSciences (UK) Ltd Terrett, Jonathan A
<120> A PROTEIN INVOLVED IN CARCINOMA
<130> P0180-WO01
<150> GB0208331.9
   <151> 2002-04-11.
<150> GB0221538.2
   <151> 2002-09-17
<160> 4
<170> PatentIn version 3.1
<210> 1
   <211> 357
   <212> PRT
   <213> Homo sapiens
<400> 1
   Met Ala Thr Thr Val Pro Asp Gly Cys Arg Asn Gly Leu Lys Ser Lys
   1               5                   10                  15
   Tyr Tyr Arg Leu Cys Asp Lys Ala Glu Ala Trp Gly Ile Val Leu Glu
               20                  25                  30
   Thr Val Ala Thr Ala Gly Val Val Thr Ser Val Ala Phe Met Leu Thr
           35                  40                  45
   Leu Pro Ile Leu Val Cys Lys Val Gln Asp Ser Asn Arg Arg Lys Met
       50                  55                  60
   Leu Pro Thr Gln Phe Leu Phe Leu Leu Gly Val Leu Gly Ile Phe Gly
   65                  70                  75                  80
   Leu Thr Phe Ala Phe Ile Ile Gly Leu Asp Gly Ser Thr Gly Pro Thr
                   85                  90                  95
   Arg Phe Phe Leu Phe Gly Ile Leu Phe Ser Ile Cys Phe Ser Cys Leu
               100                 105                 110
   Leu Ala His Ala Val Ser Leu Thr Lys Leu Val Arg Gly Arg Lys Pro
           115                 120                 125
   Leu Ser Leu Leu Val Ile Leu Gly Leu Ala Val Gly Phe Ser Leu Val
       130                 135                 140
   Gln Asp Val Ile Ala Ile Glu Tyr Ile Val Leu Thr Met Asn Arg Thr
   145                 150                 155                 160
   Asn Val Asn Val Phe Ser Glu Leu Ser Ala Pro Arg Arg Asn Glu Asp
                   165                 170                 175
   Phe Val Leu Leu Leu Thr Tyr Val Leu Phe Leu Met Ala Leu Thr Phe
               180                 185                 190
   Leu Met Ser Ser Phe Thr Phe Cys Gly Ser Phe Thr Gly Trp Lys Arg
           195                 200                 205
   His Gly Ala His Ile Tyr Leu Thr Met Leu Leu Ser Ile Ala Ile Trp
       210                 215                 220
   Val Ala Trp Ile Thr Leu Leu Met Leu Pro Asp Phe Asp Arg Arg Trp
   225                 230                 235                 240
   Asp Asp Thr Ile Leu Ser Ser Ala Leu Ala Ala Asn Gly Trp Val Phe
                   245                 250                 255
   Leu Leu Ala Tyr Val Ser Pro Glu Phe Trp Leu Leu Thr Lys Gln Arg
               260                 265                 270
   Asn Pro Met Asp Tyr Pro Val Glu Asp Ala Phe Cys Lys Pro Gln Leu
           275                 280                 285
   Val Lys Lys Ser Tyr Gly Val Glu Asn Arg Ala Tyr Ser Gln Glu Glu
       290                 295                 300
   Ile Thr Gln Gly Phe Glu Glu Thr Gly Asp Thr Leu Tyr Ala Pro Tyr
   305                 310                 315                 320
   Ser Thr His Phe Gln Leu Gln Asn Gln Pro Pro Gln Lys Glu Phe Ser
   325 330 335
   Ile Pro Arg Ala His Ala Trp Pro Ser Pro Tyr Lys Asp Tyr Glu Val
   340 345 350
   Lys Lys Glu Gly Ser
   355
<210> 2
   <211> 2302
   <212> DNA
   <213> Homo sapiens
<400> 2
   ccaaggtctc ccccagcact gaggagctcg cctgctgccc tcttgcgcgc gggaagcagc    60
   accaagttca cggccaacgc cttggcacta gggtccagaa tggctacaac agtccctgat   120
   ggttgccgca atggcctgaa atccaagtac tacagacttt gtgataaggc tgaagcttgg   180
   ggcatcgtcc tagaaacggt ggccacagcc ggggttgtga cctcggtggc cttcatgctc   240
   actctcccga tcctcgtctg caaggtgcag gactccaaca ggcgaaaaat gctgcctact   300
   cagtttctct tcctcctggg tgtgttgggc atctttggcc tcaccttcgc cttcatcatc   360
   ggactggacg ggagcacagg gcccacacgc ttcttcctct ttgggatcct cttttccatc   420
   tgcttctcct gcctgctggc tcatgctgtc agtctgacca agctcgtccg ggggaggaag   480
   cccctttccc tgttggtgat tctgggtctg gccgtgggct tcagcctagt ccaggatgtt   540
   atcgctattg aatatattgt cctgaccatg aataggacca acgtcaatgt cttttctgag   600
   ctttccgctc ctcgtcgcaa tgaagacttt gtcctcctgc tcacctacgt cctcttcttg   660
   atggcgctga ccttcctcat gtcctccttc accttctgtg gttccttcac gggctggaag   720
   agacatgggg cccacatcta cctcacgatg ctcctctcca ttgccatctg ggtggcctgg   780
   atcaccctgc tcatgcttcc tgactttgac cgcaggtggg atgacaccat cctcagctcc   840
   gccttggctg ccaatggctg ggtgttcctg ttggcttatg ttagtcccga gttttggctg   900
   ctcacaaagc aacgaaaccc catggattat cctgttgagg atgctttctg taaacctcaa   960
   ctcgtgaaga agagctatgg tgtggagaac agagcctact ctcaagagga aatcactcaa  1020
   ggttttgaag agacagggga cacgctctat gccccctatt ccacacattt tcagctgcag  1080
   aaccagcctc cccaaaagga attctccatc ccacgggccc acgcttggcc gagcccttac  1140
   aaagactatg aagtaaagaa agagggcagc taactctgtc ctgaagagtg ggacaaatgc  1200
   agccgggcgg cagatctagc gggagctcaa agggatgtgg gcgaaatctt gagtcttctg  1260
   agaaaactgt acaagacact acgggaacag tttgcctccc tcccagcctc aaccacaatt  1320
   cttccatgct ggggctgatg tgggctagta agactccagt tcttagaggc gctgtagtat  1380
   tttttttttt ttgtctcatc ctttggatac ttcttttaag tgggagtctc aggcaactca  1440
   agtttagacc cttactcttt ttgtttgttt tttgaaacag gatcttgctc tgtcacccag  1500
   gcttgagtgc agtggtgcga tcacagccca gtgcagcctc gaccacctgt gctcaagcaa  1560
   tcctcccatc tccatctccc aaagtgctgg gatgacaggc gtgagccaca gctcccagcc  1620
   taggccctta atcttgctgt tattttccat ggactaaagg tctggtcatc tgagctcacg  1680
   ctggctcaca cagctctagg ggcctgctcc tctaactcac agtgggtttt gtgaggctct  1740
   gtggcccaga gcagacctgc atatctgagc aaaaatagca aaagcctctc tcagcccact  1800
   ggcctgaatc tacactggaa gccaacttgc tggcaccccc gctccccaac ccttcttgcc  1860
   tgggtaggag aggctaaaga tcaccctaaa tttactcatc tctctagtgc tgcctcacat  1920
   tgggcctcag cagctcccca gcaccaattc acaggtcacc cctctcttct tgcactgtcc  1980
   ccaaacttgc tgtcaattcc gagatctaat ctccccctac gctctgccag gaattctttc  2040
   agacctcact agcacaagcc cggttgctcc ttgtcaggag aatttgtaga tcattctcac  2100
   ttcaaattcc tggggctgat acttctctca tcttgcaccc caacctctgt aaatagattt  2160
   accgcattta cggctgcatt ctgtaagtgg gcatggtctc ctaatggagg agtgttcatt  2220
   gtataataag ttattcacct gagtatgcaa taaagatgtg gtggccactc tttcatggtg  2280
   gtggcagcaa aaaaaaaaaa aa                                           2302
<210> 3
   <211> 22
   <212> DNA
   <213> Artificial sequence
<220>
   <223> Primer
<400> 3
   ctcgtgaaga agagctatgg tc 22
<210> 4
   <211> 22
   <212> DNA
   <213> Artificial sequence
<220>
   <223> Primer
<400> 4
   cactcttcag gacagagtta gc 22

## Description

The present invention relates to the screening for breast cancer, colon cancer and/or osteosarcoma in a subject, and/or monitoring the effectiveness of an anti-breast cancer, anti-colon cancer and/or anti-osteosarcoma therapy using the polypeptide RAIG1 (retinoic acid-inducible gene 1; also known as hypothetical protein FLJ10899 or retinoic acid induced 3) or a corresponding nucleic acid. The use of an antibody which specifically binds to a RAIG1 polypeptide in the manufacture of a medicament for the treatment of breast cancer, colon cancer and/or osteosarcoma, wherein the antibody is conjugated to a therapeutic or drug moiety, is also provided.

Tumour specific proteins have been identified for a number of cancer types using techniques such as differential screening of cDNAs (Hubert, R.S., *et al.,* 1999, *Proc. Natl. Acad, Sci. USA* 96:14523-14528) and the purification of cell-surface proteins that are recognised by tumour-specific antibodies (Catimel, B., *et al.,* 1996, J. Biol. Chem 271: 25664-25670). More recently, DNA 'chips' containing up to 10,000 expressed sequence elements have been used to characterise tumour cell gene expression (Dhanasekaran, S.M*., et al.,* 2001, Nature 412:822-826). However, there are several reasons why the numerous and extensive previous transcriptomic analysis of cancers may not have revealed all, or even most, tumour associated proteins. These include: (i) a lack of correlation between transcript and disease-associated protein levels, particularly common for membrane proteins that often have a long half-life and as such do not have a high mRNA turnover. Therefore, whilst the difference in protein levels between normal and cancerous cells are consistent it is often difficult to associate changes in the mRNA for a given membrane protein with the cancerous state. (ii) Translocation of a protein in the disease state rather than simply differential levels of the transcript, for example, erbB2/HER2-neu, shows much greater plasma-membrane localisation in cancer cells than normal breast cells, and the transcription factors oestrogen receptor and STAT3 translocate to the nucleus to exert their tumourigenic effects. (iii) Novel, uncharacterised genes are not highly represented within the 'closed system' of a cDNA array where there are restrictions on the number of expressed sequence elements per chip and the knowledge and availability of DNA clones.

Therefore, a need exists to identify further markers for the diagnosis of cancer and further targets that may be used in a therapeutic approach to cancer. The present invention is based on the novel association of RAIG1 with carcinoma, in particular breast cancer, pancreatic cancer, lung cancer, liver cancer, ovarian cancer, colon cancer and/or osteosarcoma. Analysis of RAIG1 mRNA expression revealed that it is upregulated in colon cancer, breast cancer, lung cancer, pancreatic cancer, ovarian cancer and osteosarcoma when compared to normal tissue, or matched control tissue.

EP 1074617 relates to primer sets for synthesizing full length cDNAs, useful for studying protein function, and discloses a RAIG1 nucleotide sequence as one of 16,000 sequences useful for making such primer sets. However no disease association is noted.

RAIG1 is an orphan G-protein coupled receptor (GPCR) located on chromosome 12 (Cheng & Lotan, above; Brauner-Osbourne, H., 2001, Biochim. Biophys. Acta 1518:237-248). Unlike a related receptor (GPCR5B) which is widely expressed in peripheral and central tissues, RAIG1 has been reported to show a more restricted expression pattern (Brauner-Osbourne, H. & Krogsgaard-Larsen, P. 2000, Genomics, 65:121-128).

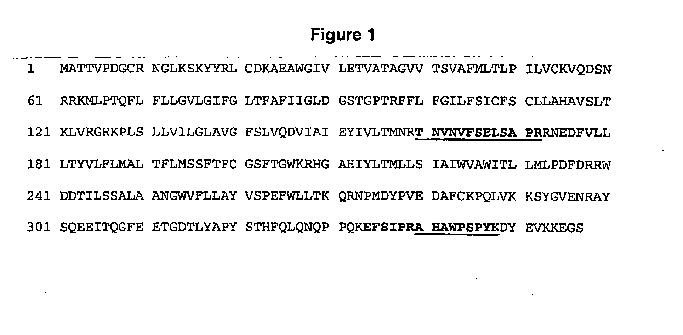

Accordingly, the present invention provides a method of screening for breast cancer, colon cancer and/or osteosarcoma in a subject, and/or monitoring the effectiveness of an anti-breast cancer, anti-colon cancer and/or anti-osteosarcoma therapy, which comprises the step of detecting and/or quantifying in a biological sample obtained from said subject a RAIG1 polypeptide which comprises or consists of the amino acid sequence shown in Figure 1 (SEQ ID NO: 1); wherein said RAIG1 polypeptide is detected and/or quantified using an antibody that specifically binds to said RAIG1 polypeptide.

In the context of the present invention, RAIG1 polypeptides can be obtained from a biological sample from any source, such as and without limitation, a sample of breast, pancreatic, lung, liver, ovarian, colon and/or bone marrow tissue. In one embodiment, the level of the RAIG1 polypeptide is compared to a reference range or control.

The term 'carcinoma' includes a malignant new growth that arises from epithelium, found in skin or, more commonly, the lining of body organs, for example: breast, prostate, lung, kidney, pancreas, stomach or bowel. Carcinomas tend to infiltrate into adjacent tissue and spread (metastasise) to distant organs, for example: to bone, liver, lung or the brain.

The polypeptides described above are hereinafter referred to as "RAIG1 polypeptides". The term "polypeptides" includes peptides, polypeptides and proteins. These are used interchangeably unless otherwise specified. RAIG1 polypeptides may be in the form of a 'mature' protein or may be part of a larger protein such as a fusion protein. It is often advantageous to include an additional amino acid sequence which contains secretory or leader sequences, a pre-, pro- or prepro-protein sequence, or a sequence which aids in purification such as an affinity tag, for example, but without limitation, multiple histidine residues, a FLAG tag, HA tag or myc tag. An additional sequence which may provide stability during recombinant production may also be used. Such sequences may be optionally removed as required by incorporating a cleavable sequence as an additional sequence or part thereof. Thus, a RAIG1 polypeptide may be fused to other moieties including other polypeptides. Such additional sequences and affinity tags are well known in the art.

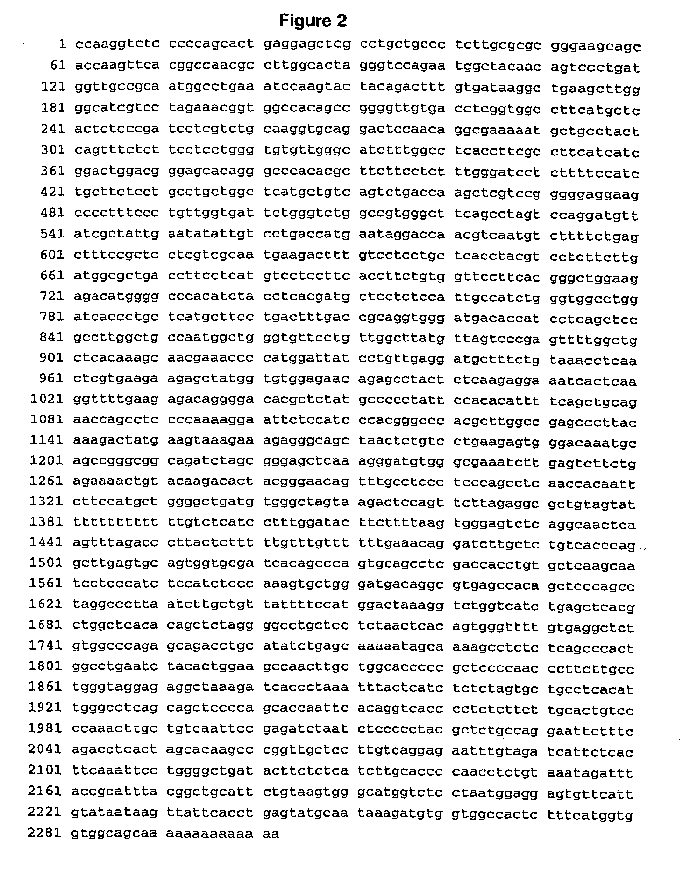

The invention also provides a method of screening for breast cancer, colon cancer and/or osteosarcoma in a subject, and/or monitoring the effectiveness of an anti-breast cancer, anti-colon cancer and/or anti-osteosarcoma therapy which comprises the step of detecting in a biological sample obtained from said subject, a nucleic acid which
a) comprises or consists of the DNA sequence shown in Figure 2 (SEQ ID NO: 2) or its RNA equivalent;
b) has a sequence which is complementary to a sequence of a); or
c) has a sequence which codes for a polypeptide as defined above.

Unless the context indicates otherwise, RAIG1 nucleic acids include those nucleic acid molecules defined in a) to c) above and may have one or more of the following characteristics:
1) they may be DNA or RNA;
2) they may be single or double stranded;
3) they may be in substantially pure form. Thus, they may be provided in a form which is substantially free from contaminating proteins and/or from other nucleic acids; and
4) they may be with introns or without introns (*e.g*. as cDNA).

The use of nucleic acids which are complementary to the RAIG1 nucleic acids described in a)-c) above, and can hybridise to said RAIGI nucleic acids is possible. Such nucleic acid molecules are referred to as "hybridising" nucleic acid molecules. For example, but without limitation, hybridising nucleic acid molecules can be useful as probes or primers. Hybridising nucleic acid molecules may have high degree of sequence identity along its length with a nucleic acid molecule within the scope of a)-c) above (*e.g.* at least 50%, at least 75%, at least 80%, at least 85%, at least 90%, at least 95%, or at least 98% sequence identity).

Hybridisation assays can be used for detection, prognosis, diagnosis, or monitoring of therapy of carcinoma, *e.g*. breast cancer, pancreatic cancer, lung cancer, liver cancer, ovarian cancer, colon cancer and/or osteosarcoma, in a subject. Accordingly, such a hybridisation assay comprises:
i) contacting a biological sample, obtained from a subject, containing nucleic acid with a nucleic acid probe capable of hybridising to a RAIG1 nucleic acid molecule, under conditions such that hybridisation can occur; and
ii) detecting or measuring any resulting hybridisation.

Preferably, such hybridising molecules are at least 10 nucleotides in length and are preferably at least 25 or at least 50 nucleotides in length. More preferably, the hybridising nucleic acid molecules specifically hybridise to nucleic acids within the scope of a)-c, above. Most preferably, the hybridisation occurs under stringent hybridisation conditions. One example of stringent hybridisation conditions is where attempted hybridisation is carried out at a temperature of from about 35°C to about 65°C using a salt solution which is about 0.9M. However, the skilled person will be able to vary such conditions as appropriate in order to take into account variables such as probe length, base composition, type of ions present, etc.

A diagnostic kit comprising a nucleic acid probe capable of hybridising to RNA encoding a RAIG1 polypeptide. suitable reagents and instructions for use can be provided.

A diagnostic kit can be provided comprising in one or more containers a pair of primers that under appropriate reaction conditions can prime amplification of at least a portion of a RAIG1 nucleic acid molecule, such as by polymerase chain reaction (see *e.g.* Innis *et al.,* 1990, PCR Protocols, Academic Press, Inc., San Diego, CA), ligase chain reaction (see EP 320,308) use of Qβ replicase, cyclic probe reaction, or other methods known in the art. Typically, primers are at least eight nucleotides long and will preferably be at least ten to twenty-five nucleotides long and more preferably fifteen to twenty-five nucleotides long. In some cases, primers of at least thirty or at least thirty-five nucleotides in length may be used.

In a further aspect, the method of detecting the presence of a RAIG1 polypeptide comprises detecting the captured polypeptide using a directly-or indirectly labelled detection reagent.

A RAIG1 polypeptide can be detected by means of any immunoassay known in the art, including, without limitation, competitive and non competitive assay systems using techniques such as Western blots, radioimmunoassays, ELISA (enzyme linked immunosorbent assay), "sandwich" immunoassays, immunoprecipitation assays, precipitin reactions, gel diffusion precipitin reactions, immunodiffusion assays, agglutination assays, complement-fixation assays, immunoradiometric assays, fluorescent immunoassays and protein A immunoassays.

Diagnostic kits, comprising a capture reagent (e.g. an antibody) against a R.AIG1 polypeptide as defined above can be provided. In addition, such a kit may optionally comprise one or more of the following:
(1) instructions for using the capture reagent for diagnosis, prognosis, therapeutic monitoring or any combination of these applications;
(2) a labelled binding partner to the capture reagent;
(3) a solid phase (such as a reagent strip) upon which the capture reagent is immobilised; and
(4) a label or insert indicating regulatory approval for diagnostic, prognostic or therapeutic use or any combination thereof.

If no labelled binding partner to the capture reagent is provided, the anti-polypeptide capture reagent itself can be labelled with a detectable marker, e.g. a chemiluminescent, enzymatic, fluorescent, or radioactive moiety (see above).

Methods of diagnosis may be performed using a number of methods known to those skilled in the art, including, without limitation, immunoprecipitation followed by sodium dodecyl sulfate polyacrylamide gel electrophoresis, 2 dimensional gel electrophoresis, competitive and non-competitive assay systems using techniques such as Western blots, immunocytochemistry, immunohistochemistry, immunoassays, *e.g*. radioimmunoassays, ELISA (enzyme linked immunosorbent assay), "sandwich" immunoassays, immunoprecipitation assays, precipitin reactions, gel diffusion precipitin reactions, immunodiffusion assays, agglutination assays, complement-fixation assays, immunoradiometric assays, fluorescent immunoassays and protein A immunoassays.

The RAIG1 polypeptides represent a suitable target for the treatment of carcinoma, *e.g.* breast cancer, pancreatic cancer, lung cancer, liver cancer, ovarian cancer, colon cancer and/or osteosarcoma. Thus methods for identifying agents that are capable of interacting with or modulating the expression or activity of a RAIGI polypeptide or the expression of a RAIG1 nucleic acid molecule are possible. Agents identified through these screening methods are potential therapeutics for use in the treatment of carcinoma, *e.g.* breast cancer, pancreatic cancer, lung cancer, liver cancer, ovarian cancer, colon cancer and/or osteosarcoma.

Agents can be selected from a wide variety of candidate agents. Examples of candidate agents include but are not limited to, nucleic acids (*e.g*. DNA and RNA), antibodies, carbohydrates, lipids, proteins, polypeptides, peptides, peptidomimetics, small molecules and other drugs. Agents can be obtained using any of the numerous approaches in combinatorial library methods known in the art, including: biological libraries; spatially addressable parallel solid phase or solution phase libraries; synthetic library methods requiring deconvolution; the "one-bead one-compound" library method; and synthetic library methods using affinity chromatography selection. The biological library approach is suited to peptide libraries, while the other four approaches are applicable to peptide, non-peptide oligomer or small molecule libraries of compounds (Lam, 1997, Anticancer Drug Des. 12:145; U.S. 5,738,996; and U.S. 5,807,683).

Examples of suitable-methods-based-on the-present-description for the synthesis of molecular libraries can be found in the art, for example in: DeWitt *et al.,* 1993, Proc. Natl. Acad. Sci. USA 90:6909; Erb *et al.,* 1994, Proc. Natl. Acad. Sci. USA 91:11422; Zuckermann *et al.,* 1994, J. Med. Chem. 37:2678; Cho *et al.,* 1993, Science 261:1303; Carrell *et al.,* 1994, Angew. Chem. Int. Ed. Engl. 33:2059; Carell *et al.,* 1994, Angew. Chem. Int. Ed. Engl. 33:2061; and Gallop *et al.,* 1994, J. Med. Chem. 37:1233.

Libraries of compounds may be presented, for example, in solution (*e.g*. Houghten, I992, Bio/Techniques 13:412-421), or on beads (Lam, 1991, Nature 354:82-84), chips (Fodor, 1993, Nature 364:555-556), bacteria (US 5,223,409), spores (US 5,571,698; 5,403,484; and 5,223,409), plasmids (Cull *et al.,* 1992, Proc. Natl. Acad. Sci. USA 89:1865-1869) or phage (Scott and Smith, 1990, Science 249:386-390; Devlin, 1990, Science 249:404-406; Cwirla *et al.* 1990, Proc. Natl. Acad. Sci. USA 87:6378-6382; and Felici, 1991, J. Mol. Biol. 222:301-310).

Agents that interact with (*e.g*. bind to) a RAIG1 polypeptide can be identified in a cell-based assay where a population of cells expressing a RAIG1 polypeptide can be contacted with a candidate agent and the ability of the candidate agent to interact with the polypeptide is determined. Preferably, the ability of a candidate agent to interact with a RAIG1 polypeptide can be compared to a reference range or control. Alternatively a first and second population of cells expressing a RAIG1 polypeptide can be contacted with a candidate agent or a control agent and the ability of the candidate agent to interact with the polypeptide can be determined by comparing the difference in interaction between the candidate agent and control agent. If desired, this type of assay may be used to screen a plurality (*e.g*. a library) of candidate agents using a plurality of cell populations expressing a RAIG1 polypeptide. If desired, this assay may be used to screen a plurality (*e.g.* a library) of candidate agents. The cell, for example, can be of prokaryotic origin (*e.g. E. coli*) or eukaryotic origin (*e.g*. yeast or mammalian). Further, the cells can express the RAIG1 polypeptide endogenously or be genetically engineered to express the polypeptide. In some embodiments, a RAIG1 polypeptide or the candidate agent is labelled, for example with a radioactive label (such as ³²P, ³⁵S or ¹²⁵I) or a fluorescent label (such as fluorescein isothiocyanate, rhodamine, phycoerythrin, phycocyanin, allophycocyanin, o-phthaldehyde or fluorescamine) to enable detection of an interaction between a polypeptide and a candidate agent.

Agents that interact with *(e.g.* bind to) a RAIG1 polypeptide can be identified in a cell-free assay system where a sample expressing a RAIG1 polypeptide is contacted with a candidate agent and the ability of the candidate agent to interact with the polypeptide is determined. Preferably, the ability of a candidate, agent to interact with a RAIG1 polypeptide can be compared to a reference range or control. Preferably, a first and second sample comprising native or recombinant RAIG1 polypeptide can be contacted with a candidate agent or a control agent and the ability of the candidate agent to interact with the polypeptide can be determined by comparing the difference in interaction between the candidate agent and control agent. If desired, this assay may be used to screen a plurality (*e.g*. a library) of candidate agents using a plurality of RAIG1 polypeptides samples. Preferably, the polypeptide is first immobilized, by, for example, contacting the polypeptide with an immobilized antibody which specifically recognizes and binds it, or by contacting a purified preparation of polypeptide with a surface designed to bind proteins. The polypeptide may be partially or completely purified (*e.g.* partially or completely free of other polypeptides) or part of a cell lysate. Further, the polypeptide may be a fusion protein comprising the RAIG1-polypeptide or a biologically active portion thereof and a domain-such as glutathionine-S-transferase. Alternatively, the polypeptide can be biotinylated using techniques well known to those of skill in the art (*e.g*. biotinylation kit, Pierce Chemicals; Rockford, IL). The ability of the candidate agent to interact with the polypeptide can be can be duplicated by methods known to those of skill in the art.

A RAIG1 polypeptide can be used as a "bait protein" in a two-hybrid assay or three hybrid assay to identify other proteins that bind to or interact with the RAIG1 polypeptide (see *e.g.* US 5,283,317; Zervos *et al*., 1993, Cell 72:223-232; Madura *et al.* 1993, J. Biol. Chem. 268:12046-12054; Bartel *et al.,* 1993, Bio/Techniques 14:920-924; Iwabuchi *et al.,* 1993, Oncogene 8:1693-1696; and WO 94/10300). As those skilled in the art will appreciate, such binding proteins are also likely to be involved in the propagation of signals by a RAIG1 polypeptide. For example, they may be upstream or downstream elements of a signalling pathway involving a RAIG1 polypeptide. Alternatively, polypeptides that interact with a RAIG1 polypeptide can be identified by isolating a protein complex comprising a RAIG1 polypeptide (*i.e.* a RAIG1 polypeptide which interacts directly or indirectly with one or more other polypeptides) and identifying the associated proteins using methods known in the art such as mass spectrometry or Western blotting (for examples see Blackstock, W. & Weir, M. 1999, Trends in Biotechnology, 17: 121-127; Rigaut, G. 1999, Nature Biotechnology, 17: 1030-1032; Husi, H. 2000, Nature Neurosci. 3:661-669; Ho, Y. *et al.,* 2002, Nature, 415:180-183; Gavin, A*. et al.,* 2002, Nature, 415: 141-147).

In all cases, the ability of the candidate agent to interact directly or indirectly with the RAIG1 polypeptide can be determined by methods known to those of skill in the art. For example but without limitation, the interaction between a candidate agent and a RAIG1 polypeptide can be determined by flow cytometry, a scintillation assay, an activity assay, mass spectrometry, microscopy, immunoprecipitation or western blot analysis.

Agent that competitively interact with (i.e. competitively bind to) a RAIG1 polypeptide can be identified in a competitive binding assay and the ability of the candidate agent to interact with the RAIG1 polypeptide can be determined. Preferably, the ability of a candidate agent to interact with a RAIG1 polypeptide can be compared to a reference range or control. Preferably a first and second population of cells expressing both a RAIG1 polypeptide and a protein which is known to interact with the RAIG1 polypeptide can be contacted with a candidate agent or a control agent. The ability of the candidate agent to competitively interact with the RAIG1 polypeptide can then be determined by comparing the interaction in the first and second population of cells. Alternatively an alternative second population or a further population of cells may be contacted with an agent which is known to competitively interact with a RAIG1 polypeptide. Alternatively, agents that competitively interact with a RAIG1 polypeptide are identified in a cell-free assay system by contacting a first and second sample comprising a RAIG1 polypeptide and a protein known to interact with the RAIG1 polypeptide with a candidate agent or a control agent. The ability of the candidate agent to competitively interact with the RAIG1 polypeptide is then determined by comparing the interaction in the first and second sample. Alternatively, an alternative second sample or a further sample comprising a RAIG1 polypeptide may be contacted with an agent which is known to competitively interact with a RAIG1 polypeptide. In any case, the RAIG1 polypeptide-and known interacting protein may be expressed naturally or may be recombinantly expressed; the candidate agent may be added exogenously, or be expressed naturally or recombinantly.

Agents that modulate an interaction between a RAIG1 polypeptide and another agent, for example but without limitation a protein, may be identified in a cell-based assay by contacting cells expressing a RAIG1 polypeptide in the presence of a known interacting agent and a candidate modulating agent and selecting the candidate agent which modulates the interaction. Alternatively, agents that modulate an interaction between a RAIG1 polypeptide and another agent, for example but without limitation a protein, may be identified in a cell-free assay system by contacting the polypeptide with an agent known to interact with the polypeptide in the presence of a candidate agent. A modulating agent can act as an antibody, a cofactor, an inhibitor, an activator or have an antagonistic or agonistic effect on the interaction between a RAIG1 polypeptide and a known agent. As stated above the ability of the known agent to interact with a RAIG1 polypeptide can be determined by methods known in the art. These assays, whether cell-based or cell-free, can be used to screen a plurality (*e.g*. a library) of candidate agents.

A cell-based assay system can be used to identify agents capable of modulating (i.e. stimulating or inhibiting) the activity of a RAIG1 polypeptide. Accordingly, the activity of a RAIG1 polypeptide is measured in a population of cells that naturally or recombinantly express a RAIG1 polypeptide, in the presence of a candidate agent. Preferably, the activity of a RAIG1 polypeptide is compared to a reference range or control. Preferably, the activity of a RAIG1 polypeptide is measured in a first and second population of cells that naturally or recombinantly express a RAIG1 polypeptide, in the presence of agent or absence of a candidate agent (*e.g*.*,* in the presence of a control agent) and the activity of the RAIG1 polypeptide is compared. The candidate agent can then be identified as a modulator of the activity of a RAIG1 polypeptide based on this comparison. Alternatively, the activity of a RAIG 1 polypeptide can be measured in a cell-free assay system where the RAIG1 polypeptide is either natural or recombinant. Preferably, the activity of a RAIG1 polypeptide is compared to a reference range or control. Preferably, the activity of a RAIGI polypeptide is measured in a first and second sample in the presence or absence of a candidate agent and the activity of the RAIG1 polypeptide is compared. The candidate agent can then be identified as a modulator of the activity of a RAIG1 polypeptide based on this comparison.

The activity of a RAIG1 polypeptide can be assessed by detecting its effect on a downstream effector, for example but without limitation, the level or activity of a second messenger (*e.g.* cAMP, intracellular Ca²⁺, diacylglycerol, IP₃, etc.), detecting catalytic or enzymatic activity, detecting the induction of a reporter gene *(e.g.* luciferase) or detecting a cellular response, for example, proliferation, differentiation or transformation where appropriate as known by those skilled in the art (for activity measurement techniques see, *e.g*. US 5,401,639). The candidate agent can then be identified as a modulator of the activity of a RAIG1 polypeptide by comparing the effects of the candidate agent to the control agent. Suitable control agents include PBS or normal saline.

Agents such as an enzyme, or a biologically active portion thereof, which is responsible for the production or degradation of a RAIG1 polypeptide or nucleic acid, or is responsible for the post-translational modification of a RAIG1 polypeptide can be identified. In a primary screen, substantially pure, native or recombinantly expressed RAIG1 polypeptides, nucleic acids or cellular extract or other sample comprising native or recombinantly expressed RAIG1 polypeptides or nucleic acids are contacted with a plurality of candidate agents (for example but without limitation, a plurality of agents presented as a library) that may be responsible for the processing of a RAIG1 polypeptide or nucleic acid, in order to identify such agents. The ability of the candidate agent to modulate the production, degradation or post-translational modification of a RAIG1 polypeptide or nucleic acid can be determined by methods known to those of skill in the art, including without limitation, flow cytometry, radiolabelling, a kinase assay, a phosphatase assay, immunoprecipitation and Western blot analysis, or Northern blot analysis.

Cells expressing a RAIG1 polypeptide can be contacted with a plurality of candidate agents. The ability of such an agent to modulate the production, degradation or post-translational modification of a RAIG1 polypeptide can be determined by methods known to those of skill in the art, as described above.

Agents that modulate the expression of a RAIG1 polypeptide (*i*.*e*. up-regulate or down-regulate) can be identified in a cell-based assay system. Accordingly, a population of cells expressing a RAIG1 polypeptide or nucleic acid are contacted with a candidate agent and the ability of the candidate agent to alter expression of the RAIG1 polypeptide or nucleic acid is determined by comparison to a reference range or control. Alternatively a first and second population of cells expressing a RAIG1 polypeptide are contacted with a candidate agent or a control agent and the ability of the candidate agent to alter the expression of the RAIG1 polypeptide or nucleic acid is determined by comparing the difference in the level of expression of the RAIG1 polypeptide or nucleic acid between the first and second populations of cells. Alternatively the expression of the RAIG1 polypeptide or nucleic acid in the first population may be further compared to a reference range or control. If desired, this assay may be used to screen a plurality (*e.g*. a library) of candidate agents. The cell, for example, can be of prokaryotic origin (*e.g. E. coli)* or eukaryotic origin (*e.g*. yeast or mammalian). Further, the cells can express a RAIG1 polypeptide or nucleic acid endogenously or be genetically engineered to express a RAIG1 polypeptide or nucleic acid. The ability of the candidate agents to alter the expression of a RAIGI polypeptide or nucleic acid can be determined by methods known to those of skill in the art, for example and without limitation, by flow cytometry, radiolabelling, a scintillation assay, immunoprecipitation, Western blot analysis or Northern blot analysis.

Agents that modulate the expression of a RAIG1 polypeptide or nucleic acid can be identified in an animal model. Examples of suitable animals include, but are not limited to, mice, rats, rabbits, monkeys, guinea pigs, dogs and cats. Preferably, the animal used represents a model of carcinoma, e.g. breast cancer, pancreatic cancer, lung cancer, liver cancer, ovarian cancer, colon cancer and/or osteosarcoma. Accordingly, a first and second group of mammals are administered with a candidate agent or a control agent and the ability of the candidate agent to modulate the expression of the RAIG1 polypeptide or nucleic acid is determined by comparing the difference in the level of expression between the first and second group of mammals. Where desired, the expression levels of the RAIG1 polypeptides or nucleic acid in the first and second groups of mammals can be compared to the level of a RAIG1 polypeptide or nucleic acid in a control group of mammals. The candidate agent or a control agent can be administered by means known in the art (*e.g.* orally, rectally or parenterally such as intraperitoneally or intravenously). Changes in the expression of a polypeptide or nucleic acid can be assessed by the methods outlined above. Particularly, a therapeutically effective agent can be identified by monitoring an amelioration or improvement in disease symptoms, to delay onset or slow progression of the disease, for example but without limitation, a reduction in tumour size. Techniques known to physicians familiar with carcinoma can be used to determine whether a candidate agent has altered one or more symptoms associated with the disease.

One skilled in the art will also appreciate that a RAIG1 polypeptide may also be used in a method for the structure-based design of an agent, in particular a small molecule which acts to modulate (*e.g*. stimulate or inhibit) the activity of said polypeptide, said method comprising:
1) determining the three-dimensional structure of said polypeptide;
2) deducing the three-dimensional structure within the polypeptide of the likely reactive or binding site(s);
3) synthesising candidate agents that are predicted to react or bind to the deduced reactive or binding site; and
4) testing whether the candidate agent is able to modulate the activity of said polypeptide.

It will be appreciated that the method described above is likely to be an iterative process.

Agents that interact with, or modulate the expression or activity of a RAIG1 polypeptide or nucleic acid, RAIG1 polypeptides, antibodies and RAIG1 nucleic acids, and uses thereof for treatments as described herein can be provided. Hereinafter, the agents, RAIG1 polypeptides and RAIG1 nucleic acids of use in treatment are referred to as 'active agents'. The term 'treatment' includes either therapeutic or prophylactic therapy. When a reference is made herein to a method of treating or preventing a disease or condition using a particular active agent or combination of agents, it is to be understood that such a reference is intended to include the use of that active agent or combination of agents in the preparation of a medicament for the treatment or prevention of the disease or condition.

Accordingly, a method for the prophylaxis and/or treatment of carcinoma, *e.g*. breast cancer, pancreatic cancer, lung cancer, liver cancer, ovarian cancer, colon cancer and/or osteosarcoma, which comprises administering to said subject a therapeutically effective amount of at least one active agent can be provided.

In order to use active agents in therapy (human or veterinary), they will normally be formulated into a pharmaceutical composition in accordance with standard pharmaceutical practice, *e.g*. by admixing the active agent and a pharmaceutically acceptable carrier. Thus, according to a further aspect of the invention there is provided a pharmaceutical composition comprising at least one active agent of the invention and a pharmaceutically acceptable carrier. The pharmaceutical compositions are particularly useful in the prevention or treatment of carcinoma. In one aspect, the pharmaceutical composition is for use as a vaccine and so any additional components will be acceptable for vaccine use. In addition, the skilled person will appreciate that one or more suitable adjuvants may be added to such vaccine preparations.

Active agents may be administered to a subject by any of the routes conventionally used for drug administration, for example they may be administered parenterally, orally, topically (including buccal, sublingual or transdermal) or by inhalation. The most suitable route for administration in any given case will depend on the particular active agent, the carcinoma involved, the subject, and the nature and severity of the disease and the physical condition of the subject.

The active agents may be administered in combination, *e.g*. simultaneously, sequentially or separately, with one or more other-therapeutically active; *e*.*g*. anti-carcinoma, agents.

The dosage to be administered of an active agent will vary according to the particular active agent, the carcinoma involved, the subject, and the nature and severity of the disease and the physical condition of the subject, and the selected route of administration; the appropriate dosage can be readily determined by a person skilled in the art. For the treatment of carcinoma in humans and animals, the dosage may range from 0.01 mg/kg to 750 mg/kg. For prophylactic use in human and animals, the dosage may range from 0.01 mg/kg to 100 mg/kg.

The compositions may contain from 0.1% by weight, preferably from 10-60% by weight, of the active agent of the invention, depending on the method of administration.

Pharmaceutical compositions may be conveniently presented in unit dose forms containing a predetermined amount of an active agent of the invention per dose. Such a unit may contain for example but without limitation, 750mg/kg to 0.1mg/kg depending on the condition being treated, the route of administration and the age, weight and condition of the subject. Preferred unit dosage compositions are those containing a daily dose or sub-dose, as recited above, or an appropriate fraction thereof, of the active agent.

It will be recognized by one of skill in the art that the optimal quantity and spacing of individual dosages of an active agent will be determined by the nature and extent of the condition being treated, the form, route and site of administration, and the particular subject being treated, and that such optimums can be determined by conventional techniques. It will also be appreciated by one of skill in the art that the optimal course of treatment, *i.e.* the number of doses of an active agent given per day for a defined number of days, can be ascertained by those skilled in the art using conventional course of treatment determination tests.

Dosage regimens are adjusted to provide the optimum desired response. For example, a single bolus may be administered, several divided doses may be administered over time or the dose may be proportionally reduced or increased as indicated by the exigencies of the therapeutic situation.

Pharmaceutically acceptable carriers for use may take a wide variety of forms depending, e.g. on the route of administration.

Compositions for oral administration may be liquid or solid. Oral liquid preparations may be in the form of, for example, aqueous or oily suspensions, solutions, emulsions, syrups or elixirs, or may be presented as a dry product for reconstitution with water or other suitable vehicle before use. Oral liquid preparations may contain suspending agents, for example sorbitol, methyl cellulose, glucose syrup, gelatin, hydroxyethyl cellulose, carboxymethyl cellulose, aluminium stearate gel or hydrogenated edible fats, emulsifying agents, for example lecithin, sorbitan monooleate, or acacia; water; non-aqueous vehicles (which may include edible oils), for example almond oil, oily esters such as glycerine, propylene glycol, or ethyl alcohol; preservatives, for example methyl or propyl p-hydroxybenzoate or sorbic acid; flavoring agents, preservatives, coloring agents and the like may be used.

In the case of oral solid preparations such as powders, capsules and tablets, carriers such as starches, sugars, microcrystalline cellulose, diluents, granulating agents, lubricants, binders, disintegrating agents, and the like may be included. Because of their ease of administration, tablets and capsules represent the most advantageous oral dosage unit form in which case solid pharmaceutical carriers are generally employed. In addition to the common dosage forms set out above, active agents may also be administered by controlled release means and/or delivery devices. Tablets and capsules may comprise conventional carriers or excipients such as binding agents for example, syrup, acacia, gelatin, sorbitol, tragacanth, or polyvinylpyrrolidone; fillers, for example lactose, sugar, maize-starch, calcium phosphate, sorbitol or glycine; tableting lubricants, for example magnesium stearate, talc, polyethylene glycol or silica; disintegrants, for example potato starch; or acceptable wetting agents such as sodium lauryl sulphate. The tablets may be coated by standard aqueous or non-aqueous techniques according to methods well known in normal pharmaceutical practice.

Pharmaceutical compositions suitable for oral administration may be presented as discrete units such as capsules, cachets or tablets, each containing a predetermined amount of the active agent, as a powder or granules, or as a solution or a suspension in an aqueous liquid, a non-aqueous liquid, an oil in water emulsion or a water-in-oil liquid emulsion. Such compositions may be prepared by any of the methods of pharmacy but all methods include the step of bringing into association the active agent with the carrier, which constitutes one or more necessary ingredients. In general, the compositions are prepared by uniformly and intimately admixing the active agent with liquid carriers or finely divided solid carriers or both, and then, if necessary, shaping the product into the desired presentation. For example, a tablet may be prepared by compression or moulding, optionally with one or more accessory ingredients.

Compressed tablets may be prepared by compressing, in a suitable machine, the active agent in a free-flowing form such as a powder or granules, optionally mixed with a binder, lubricant, inert diluent, surface active or dispersing agent. Moulded tablets may be made by moulding, in a suitable machine, a mixture of the powdered agent moistened with an inert liquid diluent. Desirably, each tablet contains from about 1 mg to about 500 mg of the active agent and each cachet or capsule contains from about 1 to about 500 mg of the active agent.

Compositions comprising an active agent may also be prepared in powder or liquid concentrate form. Conventional water soluble excipients, such as lactose or sucrose, may be incorporated in the powders to improve their physical properties. Thus, particularly suitable powders of this invention comprise 50 to 100% w/w, and preferably 60 to 80% w/w of the combination and 0 to 50% w/w and preferably 20 to 40% w/w of conventional excipients. When used in a veterinary setting such powders may be added to animal feedstuffs, for example by way of an intermediate premix, or diluted in animal drinking water.

Liquid concentrates for oral administration suitably contain a water-soluble agent combination and may optionally include a veterinarily acceptable water miscible solvent, for example polyethylene glycol, propylene glycol, glycerol, glycerol formal or such a solvent mixed with up to 30% v/v of ethanol.

Pharmaceutical compositions suitable for parenteral administration may be prepared as solutions or suspensions of the active agents of the invention in water suitably mixed with a surfactant such as hydroxypropylcellulose. Dispersions can also be prepared in glycerol, liquid polyethylene glycols, and mixtures thereof in oils. Under ordinary conditions of storage and use, these preparations contain a preservative to prevent the growth of microorganisms.

The pharmaceutical forms suitable for injectable use include aqueous or non-aqueous sterile injection solutions which may contain anti-oxidants, buffers, bacteriostats and solutes which render the composition isotonic with the blood of the intended recipient, and aqueous and non-aqueous sterile suspensions-which may include suspending agents and thickening agents. Extemporaneous injection solutions, dispersions and suspensions may be prepared from sterile powders, granules and tablets.

The compositions may be presented in unit-dose or multi-dose containers, for example in sealed ampoules and vials and to enhance stability, may be stored in a freeze-dried (lyophilized) condition requiring only the addition of the sterile liquid carrier, for example water for injections, immediately prior to use. The sterile liquid carrier may be supplied in a separate vial or ampoule and can be a solvent or dispersion medium containing, for example, water, ethanol, polyol (*e.g*, glycerol, propylene glycol and liquid polyethylene glycol), suitable mixtures thereof, and vegetable oils. Advantageously, agents such as a local anaesthetic, preservative and buffering agents can be included the sterile liquid carrier.

The active agents can be formulated to ensure proper distribution in vivo, for example, in liposomes. For methods of manufacturing liposomes, see, *e.g.* US 4,522,811; 5,374,548; and 5,399,331. The liposomes may comprise one or more moieties which are selectively transported into specific cells or organs, thus enhance targeted drug delivery (see, e.g. Ranade, V. 1989, J. Clin. Pharmacol. 29: 685).

Exemplary targeting moieties include folate or biotin (see, *e.g*., US 5,416,016); mannosides (Umezawa *et al.,* 1988, Biochem. Biophys. Res. Comm. 153:1038); antibodies (Bloeman, P. *et al.,* 1995, FEBS Lett. 357:140; Owais, M. *et al.* (1995) Antimicrob. Agents Chemother. 39: 180); surfactant protein A receptor (Briscoe *et al.* (1995) Am. J. Physiol. 1233: 134), different species of which may comprise the compositions as well as components of the active agents; psi 20 (Schreier *et al.* (1994) J. Biol. Chem. 269: 9090); see also Keinanen, K. & Laukkanen, M., 1994, FEBS Lett. 346: 123; Killion, J. & Fidler, I., 1994, Immunomethods 4: 273. The active agents can be formulated in liposomes; more preferably, the liposomes include a targeting moiety. Most preferably, the therapeutic active agents in the liposomes are delivered by bolus injection to a site proximal to the tumour.

Pharmaceutical compositions adapted for topical administration may be formulated as ointments, creams, suspensions, lotions, powders, solutions, pastes, gels, impregnated dressings, sprays, aerosols or oils, transdermal devices, dusting powders, and the like. These compositions may be prepared via conventional methods containing the active agent. Thus, they may also comprise compatible conventional carriers and additives, such as preservatives, solvents to assist drug penetration, emollients in creams or ointments and ethanol or oleyl alcohol for lotions. Such carriers may be present as from about 1% up to about 98% of the composition. More usually they will form up to about 80% of the composition. As an illustration only, a cream or ointment is prepared by mixing sufficient quantities of hydrophilic material and water, containing from about 5-10% by weight of the active agent, in sufficient quantities to produce a cream or ointment having the desired consistency.

Pharmaceutical compositions adapted for transdermal administration may be presented as discrete patches intended to remain in intimate contact with the epidermis of the recipient for a prolonged period of time. For example, the active agent may be delivered from the patch by iontophoresis.

For applications to external tissues, for example the mouth and skin, the compositions are preferably applied as a topical ointment or cream When formulated in an ointment, the active agent may be employed with either a paraffinic or a water-miscible ointment base. Alternatively, the active agent may be formulated in a cream with an oil-in-water cream base or a water-in-oil base.

Pharmaceutical compositions adapted for topical administration in the mouth include lozenges, pastilles and mouth washes.

Pharmaceutical compositions adapted for topical administration to the eye include eye drops wherein the active agent is dissolved or suspended in a suitable carrier, especially an aqueous solvent. They also include topical ointments or creams as above.

Pharmaceutical compositions suitable for rectal administration wherein the carrier is a solid are most preferably presented as unit dose suppositories. Suitable carriers include cocoa butter or other glyceride or materials commonly used in the art, and the suppositories may be conveniently formed by admixture of the combination with the softened or melted carrier(s) followed by chilling and shaping moulds. They may also be administered as enemas.

Pharmaceutical compositions adapted for vaginal administration may be presented as pessaries, tampons, creams, gels, pastes, foams or spray compositions. These may comprise emollients or bases as commonly used in the art.

The use of at least one RAIG1 polypeptide, in the preparation of a pharmaceutical composition for use in the treatment of carcinoma, *e.g*. breast cancer, pancreatic cancer, lung cancer, liver cancer, ovarian cancer, colon cancer and/or osteosarcoma can be provided. Preferably, recombinant RAIG1 polypeptides can be used in the manufacture of a pharmaceutical composition for the treatment of carcinoma, *e.g*. breast cancer, pancreatic cancer, lung cancer, liver cancer, ovarian cancer, colon cancer and/or osteosarcoma. Particularly, a RAIG1 polypeptide is fused to another polypeptide, such as the protein transduction domain of the HIV/Tat protein, which facilitates the entry of the fusion protein into a cell (Asoh, S. *et al.*, 2002, Proc. Natl. Acad. Sci. USA, 99:17107-17112) is provided for use in the manufacture of a pharmaceutical composition for the treatment of carcinoma, *e.g*. breast cancer, pancreatic cancer, lung cancer, liver cancer, ovarian cancer, colon cancer and/or osteosarcoma.

Recombinant RAIG1 polypeptides may be prepared by processes well known in the art from genetically engineered host cells comprising expression systems. Accordingly, the present invention also relates to expression systems which comprise a RAIG1 polypeptide or RAIG1 nucleic acid, to host cells which are genetically engineered with such expression systems and to the production of RAIG1 polypeptides by recombinant techniques. Cell-free translation systems systems can also be employed to produce recombinant polypeptides *(e.g*. rabbit reticulocyte lysate, wheat germ lysate, SP6/T7 *in vitro* T&T and RTS 100 *E*. *Coli* HY transcription and translation kits from Roche Diagnostics Ltd., Lewes, UK and the TNT Quick coupled Transcription/Translation System from Promega UK, Southampton, UK.

For recombinant RAIG1 polypeptide production, host cells can be genetically engineered to incorporate expression systems or portions thereof for RAIG1 nucleic acids. Such incorporation can be performed using methods well known in the art, such as, calcium phosphate transfection, DEAD-dextran mediated transfection, transvection, microinjection, cationic lipid-mediated transfection, electroporation, transduction, scrape loading, ballistic introduction or infection (see *e.g*. Davis *et al.,* Basic Methods in Molecular Biology, 1986 and Sambrook *et al.,* Molecular Cloning: A Laboratory Manual, 2^{nd} Ed., Cold Spring Harbour laboratory Press, Cold Spring Harbour, NY, 1989).

Representative examples of host cells include bacterial cells *e.g. E. Coli, Streptococci, Staphylococci, Streptomyces* and *Bacillus subtilis* cells; fungal cells, such as yeast cells and *Aspergillus* cells; insect cells such as *Drosophila* S2 and *Spodoptera* Sf9 cells; animal cells such as CHO, COS, HeLa, C127, 3T3,HEK 293, BHK and Bowes melanoma cells; and plant cells.

A wide variety of expression systems can be used, such as and without limitation, chromosomal, episomal and virus-derived systems, *e.g.* vectors derived from bacterial plasmids, from bacteriophage, from transposons, from yeast episomes, from insertion elements, from yeast chromosomal elements, from viruses such as baculoviruses, papova viruses such as SV40, vaccinia viruses, adenoviruses, fowl pox viruses, pseudorabies viruses and retroviruses, and vectors derived from combinations thereof, such as those derived from plasmid and bacteriophage genetic elements, such as cosmids and phagemids. The expression systems may contain control regions that regulate as well as engender expression. Generally, any system or vector which is able to maintain, propagate or express a nucleic acid to produce a polypeptide in a host may be used. The appropriate nucleic acid sequence may be inserted into an expression system by any variety of well-known and routine techniques, such as those set forth in Sambrook *et al., supra.* Appropriate secretion signals may be incorporated into the RAIG1 polypeptide to allow secretion of the translated protein into the lumen of the endoplasmic reticulum, the periplasmic space or the extracellular environment. These signals may be endogenous to the RAIG1 polypeptide or they may be heterologous signals.

RAIG1 polypeptides can be provided in isolated form and include RAIG1 polypeptides that have been purified to at least some extent. RAIG1 polypeptides can be produced using recombinant methods, synthetically produced or produced by a combination of these methods. RAIG1 polypeptides may be provided in substantially pure form, that is to say free, to a substantial extent, from other proteins. Thus, a RAIG1 polypeptide may be provided in a composition in which it is the predominant component present *(i.e.* it is present at a level of at least 50%; preferably at least 75%, at least 80%, at least 85%, at least 90%, or at least 95%; when determined on a weight/weight basis excluding solvents or carriers).

If a RAIG1 polypeptide is to be expressed for use in cell-based screening assays, it is preferred that the polypeptide be produced at the cell surface. In this event, the cells may be harvested prior to use in the screening assay. If the RAIG1 polypeptide is secreted into the medium, the medium can be recovered in order to isolate said polypeptide. If produced intracellularly, the cells must first be lysed before the RAIG1 polypeptide is recovered.

RAIG1 polypeptides can be recovered and purified from recombinant cell cultures by well-known methods including, ammonium sulphate or ethanol precipitation, acid extraction, anion or cation exchange chromatography, phosphocellulose chromatography, affinity chromatography, hydrophobic interaction chromatography, hydroxylapatite chromatography, molecular sieving chromatography, centrifugation methods, electrophoresis methods and lectin chromatography. A combination of the methods is used. Alternatively high performance liquid chromatography is used. Alternatively, an antibody which specifically binds to a RAIG1 polypeptide can be used to deplete a sample comprising a RAIG1 polypeptide of said polypeptide or to purify said polypeptide. Techniques well-known in the art, may be used for refolding to regenerate native or active conformations of the RAIG1 polypeptides when the polypeptides have been denatured during isolation and or purification.

The use of at least one RAIG nucleic acid in the preparation of a pharmaceutical composition for use in the treatment of carcinoma, *e.g.* breast cancer, pancreatic cancer, lung cancer, liver cancer, ovarian cancer, colon cancer and/or osteosarcoma can be provided.

Hybridising RAIG1 nucleic acid molecules can be used as anti-sense molecules, to alter the expression of RAIG1 polypeptides by binding to complementary RAIG1 nucleic acids and can be used in the treatment or prevention of carcinoma, *e.g*. breast cancer, pancreatic cancer, lung cancer, liver cancer, ovarian cancer, colon cancer and/or osteosarcoma. An antisense nucleic acid includes a RAIG1 nucleic acid capable of hybridising by virtue of some sequence complementarity to a portion of an RNA (preferably mRNA) encoding a RAIG1 polypeptide. The antisense nucleic acid can be complementary to a coding and/or non-coding region of an mRNA encoding such a polypeptide. Most preferably, expression of a RAIG1 polypeptide is inhibited by use of antisense nucleic acids. Thus, the therapeutic or prophylactic use of nucleic acids comprising at least eight nucleotides that are antisense to a gene or cDNA encoding a RAIG1 polypeptide can be provided.

Symptoms of carcinoma may be ameliorated by decreasing the level or activity of a RAIG1 polypeptide by using gene sequences encoding a polypeptide as defined herein in conjunction with well-known gene "knock-out," ribozyme or triple helix methods to decrease gene expression of the polypeptide. In this approach, ribozyme or triple helix molecules are used to modulate the activity, expression or synthesis of the gene, and thus to ameliorate the symptoms of carcinoma. Such molecules may be designed to reduce or inhibit expression of a mutant or non-mutant target gene. Techniques for the production and use of such molecules are well known to those of skill in the art.

Endogenous RAIG1 polypeptide expression can also be reduced by inactivating or "knocking out" the gene encoding the polypeptide, or the promoter of such a gene, using targeted homologous recombination *(e.g.* see Smithies, *et al.,* 1985, Nature 317:230-234; Thomas & Capecchi, 1987, Cell. 51:503-512; Thompson *et al.,* 1989, Cell 5:313-321; and Zijlstra *et al.,* 1989, Nature 342:435-438). For example, a mutant gene encoding a non-functional polypeptide (or a completely unrelated DNA sequence) flanked by DNA homologous to the endogenous gene (either the coding regions or regulatory regions of the gene encoding the polypeptide) can be used, with or without a selectable marker and/or a negative selectable marker, to transfect cells that express the target gene *in vivo.* Insertion of the DNA construct, via targeted homologous recombination, results in inactivation of the target gene.

The nucleic acid can be administered via gene therapy (see for example Hoshida, T. *et al.,* 2002, Pancreas, 25:111-121; Ikuno; Y. 2002, Invest. Ophthalmol. Vis. Sci. 2002 43:2406-2411; Bollard, C., 2002, Blood 99:3179-3187; Lee E., 2001, Mol. Med. 7:773-782). Gene therapy refers to administration to a subject of an expressed or expressible nucleic acid. Any of the methods for gene therapy available in the art can be used according to the present invention. In a one aspect, the pharmaceutical composition comprises a RAIG1 nucleic acid, said nucleic acid being part of an expression vector that expresses a RAIG1 polypeptide or chimeric protein thereof in a suitable host. In particular, such a nucleic acid has a promoter operably linked to the polypeptide coding region, said promoter being inducible or constitutive (and, optionally, tissue-specific). Alternatively, a nucleic acid molecule is used in which the coding sequences and any other desired sequences are flanked by regions that promote homologous recombination at a desired site in the genome, thus providing for intrachromosomal expression of the nucleic acid (Koller & Smithies, 1989, *Proc. Natl. Acad. Sci. USA* 86:8932-8935; Zijlstra *et al.,* 1989, Nature 342:435-438).

Delivery of the RAIG1 nucleic acid into a patient may be direct, in which case the patient is directly exposed to the nucleic acid or nucleic acid-carrying vector; this approach is known as *in vivo* gene therapy. Alternatively, delivery of the nucleic acid into the patient may be indirect, in which case cells are first-transformed with the nucleic acid *in vitro* and then transplanted into the patient; this approach is known as *ex vivo* gene therapy.

RAIG1 nucleic acids may be obtained using standard cloning and screening techniques, from a cDNA library derived from mRNA in human cells, using expressed sequence tag (EST) analysis (Adams, M*. et al.,* 1991, Science, 252:1651-1656; Adams, M. *et al.,* 1992, Nature 355:632-634; Adams, M*. et al.,* 1995, Nature, 377:Suppl: 3-174). RAIG1 nucleic acids can also be obtained from natural sources such as genomic DNA libraries or can be synthesized using well known and commercially available techniques. The RAIG1 nucleic acids comprising coding sequence for RAIG1 polypeptides described above can be used for the recombinant production of said polypeptides. The RAIG1 nucleic acids may include the coding sequence for the mature polypeptide, by itself; or the coding sequence for the mature polypeptide in reading frame with other coding sequences, such as those encoding a leader or secretory sequence, a pre-, pro- or prepro-protein sequence, a cleavable sequence or other fusion peptide portions, such as an affinity tag or an additional sequence conferring stability during production of the polypeptide. Preferred affinity tags include multiple histidine residues (for example see Gentz *et al.,* 1989, Proc. Natl. Acad. Sci USA 86:821-824), a FLAG tag, HA tag or myc tag. The RAIG1 nucleic acids may also contain non-coding 5' and 3' sequences, such as transcribed, non-translated sequences, splicing and polyadenylation signals, ribosome binding sites and sequences that stabilize mRNA.

RAIG1 polypeptide derivatives as referred to in part b) above can be created by introducing one or more nucleotide substitutions, additions or deletions into the nucleotide sequence of a RAIG1 nucleic acid such that one or more amino acid substitutions, additions or deletions are introduced into the encoded protein. - Standard techniques known to those of skill in the art can be used to introduce mutations, including, for example, site-directed mutagenesis and PCR-mediated mutagenesis. Preferably, conservative amino acid substitutions are made at one or more predicted non-essential amino acid residues.

A RAIG1 nucleic acid encoding a RAIG1 polypeptide, including homologues and orthologues from species other than human, may be obtained by a process which comprises the steps of screening an appropriate library under stringent hybridisation conditions with a labelled probe having the sequence of a RAIG1 nucleic acid as described in a)-c) above, and isolating full-length cDNA and genomic clones containing said nucleic acid sequence. Such hybridisation techniques are well-known in the art. One example of stringent hybridisation conditions is where attempted hybridisation is carried out at a temperature of from about 35°C to about 65°C using a salt solution of about 0.9M. However, the skilled person will be able to vary such conditions as appropriate in order to take into account variables such as probe length, base composition, type of ions present, etc. For a high degree of selectivity, relatively stringent conditions such as low salt or high temperature conditions, are used to form the duplexes. Highly stringent conditions include hybridisation to filter-bound DNA in 0.5M NaHPO₄, 7% sodium dodecyl sulphate (SDS), 1mM EDTA at 65°C, and washing in 0.1xSSC/0.1% SDS at 68°C (Ausubel F.M. *et al.,* eds., 1989, Current Protocols in Molecular Biology, Vol. I, Green Publishing Associates, Inc., and John Wiley & Sons, Inc., New York, at p. 2.10.3). For some applications, less stringent conditions for duplex formation are required. Moderately stringent conditions include washing in 0.2xSSC/0.1% SDS at 42°C (Ausubel *et al.,* 1989, *supra).* Hybridisation conditions can also be rendered more stringent by the addition of increasing amounts of formamide, to destabilise the hybrid duplex. Thus, particular hybridisation conditions can be readily manipulated, and will generally be chosen as appropriate. In general, convenient hybridisation temperatures in the presence of 50% formamide are: 42°C for a probe which is 95-100% identical to the fragment of a gene encoding a polypeptide as defined herein, 37°C for 90-95% identity and 32°C for 70-90% identity.

One skilled in the art will understand that, in many cases, an isolated cDNA sequence will be incomplete, in that the region coding for the polypeptide is cut short at the 5' end of the cDNA. This is a consequence of reverse transcriptase, an enzyme with inherently low processivity (a measure of the ability of the enzyme to remain attached to the template during the polymerization reaction), failing to complete a DNA copy of the mRNA template during 1^{st} strand cDNA synthesis.

Methods to obtain full length cDNAs or to extend short cDNAs are well known in the art, for example RACE (Rapid amplification of cDNA ends; *e.g.* Frohman *et al.,* 1988, Proc. Natl. Acad. Sci USA 85:8998-9002). Recent modifications of the technique, exemplified by the Marathon^{™} technology (Clontech Laboratories Inc.) have significantly simplified the search for longer cDNAs. This technology uses cDNAs prepared from mRNA extracted from a chosen tissue followed by the ligation of an adaptor sequence onto each end. PCR is then carried out to amplify the missing 5'end of the cDNA using a combination of gene specific and adaptor specific oligonucleotide primers. The PCR reaction is then repeated using nested primers which have been designed to anneal with the amplified product, typically an adaptor specific primer that anneals further 3' in the adaptor sequence and a gene specific primer that anneals further 5' in the known gene sequence. The products of this reaction can then be analysed by DNA sequencing and a full length cDNA constructed either by joining the product directly to the existing cDNA to give a complete sequence, or carrying out a separate full length PCR using the new sequence information for the design of the 5' primer.

A vaccine composition of use in the treatment of carcinoma, e.g. breast cancer, pancreatic cancer, lung cancer, liver cancer, ovarian cancer, colon cancer and/or osteosarcoma can be provided. A RAIG1 polypeptide or nucleic acid as described above can be used in the production of vaccines for treatment of carcinoma, *e.g*. breast cancer, pancreatic cancer, lung cancer, liver cancer, ovarian cancer, colon cancer and/or osteosarcoma. Such material can be antigenic and/or immunogenic. Antigenic includes a protein or nucleic acid that is capable of being used to raise antibodies or indeed is capable of inducing an antibody response in a subject. Immunogenic material includes a protein or nucleic acid that is capable of eliciting an immune response in a subject. Thus, in the latter case, the protein or nucleic acid may be capable of not only generating an antibody response but, in addition, a non-antibody based immune responses *i.e.* a cellular or humoral response. It is well known in the art that is possible to identify those regions of an antigenic or immunogenic polypeptide that are responsible for the antigenicity or immunogenicity of said polypeptide *i.e.* an epitope or epitopes. Amino acid and peptide characteristics well known to the skilled person can be used to predict the antigenic index (a measure of the probability that a region is antigenic) of a RAIG1 polypeptide. For example, but without limitation, the 'Peptidestructure' program (Jameson and Wolf, 1988, CABIOS, 4(1):181) and a technique referred to as 'Threading' (Altuvia Y. *et al.,* 1995, J. Mol. Biol. 249:244) can be used. Thus, the RAIG1 polypeptides may include one or more such epitopes or be sufficiently similar to such regions so as to retain their antigenic/immunogenic properties.

Since a polypeptide or a nucleic acid may be broken down in the stomach, the vaccine composition can preferably be administered parenterally (*e*.*g*. subcutaneous, intramuscular, intravenous or intradermal injection).

Accordingly
a) the use of such a vaccine in inducing an immune response in subject; and
b) a method for the treatment of carcinoma, *e.g.* breast cancer, pancreatic cancer, lung cancer, liver cancer, ovarian cancer, colon cancer and/or osteosarcoma, in a subject, or of vaccinating a subject against carcinoma, e.g. breast cancer, pancreatic cancer, lung cancer, liver cancer, ovarian cancer, colon cancer and/or osteosarcoma, which comprises the step of administering to the subject an effective amount of a RAIG1 polypeptide or nucleic acid, preferably as a vaccine can be provided.

A method for the treatment of breast cancer, colon cancer and/or osteosarcoma in a subject comprising administering to said subject, a therapeutically effective amount of at least one antibody that specifically bind to a RAIG1 polypeptide, wherein the antibody is conjugated to a therapeutic or drug moiety, can be provided. Antibodies which specifically bind to RAIG1 polypeptides may be used to inhibit the activity of said polypeptides.

Accordingly, there is provided the use of an antibody which specifically recognises a RAIG1 polypeptide for use in the preparation of a pharmaceutical composition for use in the treatment of breast cancer, colon cancer and/or osteosarcoma, wherein the antibody is conjugated to a therapeutic or drug moiety.

An antibody conjugated to a therapeutic moiety, can be used as a therapeutic composition that is administered alone or in combination with a cytotoxic factor(s) and/or cytokine(s). In particular, antibodies of the invention are conjugated to a therapeutic agent or drug moiety to modify a given biological response. The therapeutic agent or drug moiety is not to be construed as limited to classical chemical therapeutic agents. For example, the drug moiety may be a protein or polypeptide possessing a desired biological activity. Such moieties may include, for example and without limitation, a toxin such as abrin, ricin A, pseudomonas exotoxin, or diphtheria toxin; a protein such as tumour necrosis factor, α-interferon, β-interferon, nerve growth factor, platelet derived growth factor, tissue plasminogen activator, a thrombotic agent or an anti-angiogenic agent, *e.g.* angiostatin or endostatin; or, a biological response modifier such as a lymphokine, interleukin-1 (IL-1), interleukin-2 (IL-2), interleukin-6 (IL-6), granulocyte macrophage colony stimulating factor (GM-CSF), granulocyte colony stimulating factor (G-CSF), nerve growth factor (NGF) or other growth factor. Other therapeutic moieties may include radionuclides such as ¹¹¹In and ⁹⁰Y; antibiotics, *e.g*. calicheamicin; or drugs such as but not limited to, alkylphosphocholines, topoisomerase I inhibitors, taxoids and suramin.

Techniques for conjugating such therapeutic moieties to antibodies are well known in the art (see, e.g. Amon *et al.,* "Monoclonal Antibodies For Immunotargeting Of Drugs In Cancer Therapy", in Monoclonal Antibodies And Cancer Therapy, Reisfeld *et al.* eds., 1985 pp. 243-56, ed. Alan R. Liss, Inc; Hellstrom *et al.,* "Antibodies For Drug Delivery", in Controlled Drug Delivery, 2nd Ed., Robinson *et al.* eds., 1987, pp. 623-53, Marcel Dekker, Inc.; Thorpe, "Antibody Carriers Of Cytotoxic Agents In Cancer Therapy: A Review", in Monoclonal Antibodies '84: Biological And Clinical Applications; Pinchera *et al.,* 1985, eds., pp. 475-506; "Analysis, Results, And Future Prospective Of The Therapeutic Use Of Radiolabelled-Antibody-In Cancer Therapy", in Monoclonal Antibodies For Cancer Detection And Therapy, Baldwin *et al.* (eds.), 1985, pp. 303-16, Academic Press; Thorpe *et al.,* 1982 "The Preparation And Cytotoxic Properties Of Antibody-Toxin Conjugates", Immunol. Rev., 62:119-58 and Dubowchik *et al.,* 1999, Pharmacology and Therapeutics, 83, 67-123).

An antibody can be conjugated to a second antibody to form an antibody heteroconjugate (see US 4,676,980).

Fusion proteins of the antibodies (or functionally active fragments thereof), for example but without limitation, where the antibody or fragment thereof is fused via a covalent bond (*e.g*. a peptide bond), at optionally the N-terminus or the C-terminus, to an amino acid sequence of another protein (or portion thereof; preferably at least a 10, 20 or 50 amino acid portion of the protein) can be provided. Preferably the antibody, or fragment thereof, is linked to the other protein at the N-terminus of the constant domain of the antibody. As stated above, such fusion proteins may facilitate depletion or purification of a polypeptide as described herein, increase half-life *in vivo*, and enhance the delivery of an antigen across an epithelial barrier to the immune system.

RAIG1 polypeptides or cells expressing them can be used to produce antibodies, *e.g*. which specifically recognise said RAIG1 polypeptides. Specifically recognising or binding specifically means that the antibodies have a greater affinity for RAIG1 polypeptides than for other polypeptides. Antibodies generated against a RAIG1 polypeptide may be obtained by administering the polypeptides to an animal, preferably a non-human animal, using well-known and routine protocols.

In a further embodiment, the present invention provides the use of an antibody that specifically binds to at least one RAIG1 polypeptide for screening for breast cancer, colon cancer and/or osteosarcoma in a subject or for monitoring the efficacy of an anti-breast cancer, anti-colon cancer and/or anti-osteosarcoma therapy.

RAIG1 antibodies can be used, *inter alia*, for the diagnosis of carcinoma, *e.g*. breast cancer, pancreatic cancer, lung cancer, liver cancer, ovarian cancer, colon cancer and/or osteosarcoma, by detecting RAIG1 expression in human tissue and/or in subfractions thereof, for example but without limitation, membrane, cytosolic or nuclear subfractions.

RAIG1 antibodies include functionally active fragments, derivatives or analogues and may be, but are not limited to, polyclonal, monoclonal, bispecific, humanized or chimeric antibodies, single chain antibodies, Fab fragments and F(ab') fragments, fragments produced by a Fab expression library, anti-idiotypic (anti-Id) antibodies, and epitope-binding fragments of any of the above. Humanized antibodies are antibody molecules from non-human species having one or more complementarity determining regions (CDRs) from the non-human species and a framework region from a human immunoglobulin molecule (see, *e.g*. US 5,585,089). Antibodies include immunoglobulin molecules and immunologically active portions of immunoglobulin molecules i.e. molecules that contain an antigen binding site that specifically binds an antigen. The immunoglobulin molecules of the invention can be of any class *(e.g.* IgG, IgB, IgM, IgD and IgA) or subclass of immunoglobulin molecule.

Monoclonal antibodies may be prepared by any method known in the art such the hybridoma technique (Kohler & Milstein, 1975, Nature, 256:495-497), the trioma technique, the human B-cell hybridoma technique (Kozbor *et al.,* 1983, Immunology Today, 4:72) and the EBV-hybridoma technique (Cole *et al*., Monoclonal Antibodies and Cancer Therapy, pp77-96, Alan R Liss, Inc., 1985).

Chimeric antibodies are those antibodies encoded by immunoglobulin genes that have been genetically engineered so that the light and heavy chain genes are composed of immunoglobulin gene segments belonging to different species. These chimeric antibodies are likely to be less antigenic. Bispecific antibodies may be made by methods known in the art (Milstein *et al.,* 1983, Nature 305:537-539; WO 93/08829, Traunecker *et al.,* 1991, EMBO J. 10:3655-3659).

The antibodies for use in the present invention can also be generated using various phage display methods known in the art and include those disclosed by Brinkman *et al.* (in J. Immunol. Methods, 1995, 182: 41-50), Ames *et al.* (in J. Immunol. Methods, 1995, 184:177-186), Kettleborough *et al.* (in Eur. J. Immunol. 1994, 24:952-958), Persic *et al.* (Gene, 1997 187 9-18), Burton *et al.,* (in Advances in Immunology, 1994, 57:191-280) and in WO 90/02809; WO 91/10737; WO 92/01047; WO 92/18619; WO 93/11236; WO 95/15982; WO 95/20401; and US 5,698,426; 5,223,409; 5,403,484; 5,580,717; 5,427,908; 5,750,753; 5,821,047; 5,571,698; 5,427,908; 5,516,637; 5,780,225; 5,658,727; 5,733,743 and 5,969,108. Techniques for the production of single chain antibodies, such as those described in US 4,946,778 can also be adapted to produce single chain antibodies to NKCC1 polypeptides. Also, transgenic mice, or other organisms, including other mammals, may be used to express humanized antibodies.

Detection of the interaction of an antibody with an antigen can be facilitated by coupling the antibody to a detectable substance for example, but without limitation, an enzyme (such as horseradish peroxidase, alkaline phosphatase, beta-galactosidase, acetylcholinesterase), a prosthetic group (such as streptavidin, avidin, biotin), a fluorescent material (such as umbelliferone, fluorescein, fluorescein isothiocyanate, rhodamine, dichlorotriazinylamine fluorescein, dansyl chloride, phycoerythrin), a luminescent material (such as luminol), a bioluminescent material (such as luciferase, luciferin, aequorin), a radioactive nuclide (such as ¹²⁵I, ¹³¹I, ¹¹¹In, ⁹⁹Tc) a positron emitting metal or a non-radioactive paramagnetic metal ion (see US 4,741,900).

The antibodies of the invention include analogues and derivatives that are modified, for example but without limitation, by the covalent attachment of any type of molecule. Preferably, said attachment does not impair immunospecific binding.

In summary, the invention further provides:
(iv) the use of an-antibody that specifically binds to a RAIG 1 polypeptide in the manufacture of a medicament for the treatment of breast cancer, colon cancer and/or osteosarcoma wherein the antibody is conjugated to a therapeutic or drug moiety.

The invention will now be described with reference to the following examples, which are merely illustrative and should not in any way be construed as limiting the scope of the present invention. The examples refer to the figures in which:
**Figure 1** shows the protein sequence of RAIG1 (RAIG1; AAC98506/O95357), SEQ ID NO: 1. The tandem mass spectrum peptides are bold, underlined typeface, MALDI mass spectra peptides are bold typeface.
**Figure 2** shows the nucleic acid sequence of RAIG1 (RAIG1; AF095448), SEQ ID NO: 2.
**Figure 3** shows the distribution of RAIG1 mRNA in patient matched adjacent normal (a number followed by the letter N) and tumour breast tissues (a number followed by the letter T); mRNA levels were quantified by real time RT-PCR and are expressed as the number of copies ng⁻¹ cDNA.
**Figure 4** shows the distribution of RAIG1 mRNA in matched normal (a number followed by the letter N) and tumour colon tissues (a number followed by the letter T); mRNA levels were quantified by real time RT-PCR and are expressed as the number of copies ng⁻¹ cDNA.
**Figure 5** shows the distribution of RAIG1 mRNA in 3 matched normal (norm) and 8 pancreatic tumour tissue samples; mRNA levels were quantified by real time RT-PCR and are expressed as the number of copies ng⁻¹ cDNA.
**Figure 6** shows the distribution of RAIG1 mRNA in breast tumour tissues. 40 tumour samples were obtained from patients with (black bars) or without (white bars) lymph node metastasis, 2 normal breast tissue samples are also shown (grey bars). mRNA levels were quantified by real time RT-PCR and are expressed as the number of copies ng⁻¹ cDNA.
**Figure 7** shows the distribution of RAIG1 mRNA in normal lung tissue and 6 lung cancer samples; mRNA levels were quantified by real time RT-PCR and are expressed as the number of copies ng⁻¹ cDNA.
**Figure 8** shows the distribution of RAIG1 mRNA in normal ovary and bone marrow tissue, ovarian and osteosarcoma cell lines, 5 ovary serous cystadenocarcinoma samples, 6 ovary adenocarcinoma samples and 3 osteosarcoma samples; mRNA levels were quantified by real time RT-PCR and are expressed as the number of copies ng⁻¹ cDNA.

### Example 1 - Isolation of RAIG1 protein from breast, kidney, pancreas and liver cell lines:

Proteins in breast; kidney, -pancreas and-liver cancer cell line membranes were separated by SDS-PAGE and analysed.

### Crude fractionation of cell lines

### 1a - cell culture

Pancreatic tumour cell line HPAFII was cultured in EMEM + 2 mM Glut + 1mM NaPyr + 1%NEAA + 10% FBS + 1.5g/l Na Bicarb. Pancreatic tumour cell line Capan2 was cultured in McCoy's + 2 mM glutamine + 10% FBS + 1.5g/l NaBicarb (Capan2).

Breast cancer cell lines T47D and MCF7pool were cultured in DMF12 media containing 10% Foetal calf serum, 2mM glutamine, and 1% penicillin/streptomycin.

Hepatic cancer cell lines SK 3B2.1-7 and SKHeplpool) were cultured in EMEM + 2 mM Glut + 1mM NaPyr + 1%NEAA + 10% FBS.

Renal cancer cell lines used were CAK12 + A498 +SW839+CAKI2 pool. CAKI2 was cultured in McCoy's + 2 mM Glut + 10% FBS, A498 and SW839 cells were cultured in DMEM + 2 mM Glut + 10% FBS. All cells were grown at 37°C in a humidified atmosphere of 95% air and 5% carbon dioxide.

### 1b - Cell fractionation and plasma membrane generation

Purified membrane preparations were isolated from the cell lines. Adherent cells (2 x 10⁸) were washed three times with PBS and scraped using a plastic cell lifter. Cells were centrifuged at 1000 x g for 5min at 4°C and the cell pellet was resuspended in homogenisation buffer (250 mM sucrose, 10mM HEPES, 1mM EDTA, 1mM vanadate and 0.02% azide, protease inhibitors). Cells were fractionated using a ball bearing homogeniser (8.002 mm ball, HGM Lab equipment) until approx. 95% of cells were broken. Membranes were fractionated using the method described by Pasquali, C. *et al* .(1999, J. Chromatography 722: pp 89-102). The fractionated cells were centrifuged at 3000 x g for 10 min at 4°C and the postnuclear supernatant was layered onto a 60% sucrose cushion and centrifuged at 100 000 x g for 45 min. The membranes were collected using a pasteur pipette and layered on a preformed 15 to 60% sucrose gradient and spun at 100 000 x g for 17 hours. Proteins from the fractionated sucrose gradient were run on a 4-20% 1D-gel (Novex) and subject to western blotting; those fractions containing alkaline phosphatase and transferrin immunoreactivity but not oxidoreductase II or calnexin immunoreactivity were pooled and represented the plasma membrane fraction.

### 1c - Preparation of plasma membrane fractions for 1D-gel analysis

Plasma membrane fractions that had transferrin immunoreactivity but no oxidoreductase II or calnexin immunoreactivity were identified. These sucrose fractions were pooled and diluted at least four times with 10mM HEPES, 1mM EDTA 1mM Vanadate, 0.02% Azide. The diluted sucrose fraction was added to a SW40 or SW60 tube and centrifuged at 100 000 x g for 45min with slow acceleration and deceleration. The supernatant was removed from the membrane pellet and the pellet washed three times with PBS-CM. The membrane pellet was solubilised in 2% SDS in 63mM TrisHCl, pH 7.4. A protein assay was performed followed by the addition of mercaptoethanol (2% final), glycerol (10%) and bromophenol blue (0.0025% final) was added. A final protein concentration of 1 1µg/µl was used for 1D-gel loading.

### 1d - 1D-gel technology

Protein or membrane pellets were solubilised in 1D-sample buffer (approximately 1mg/ml) and the mixture heated to 95°C for 5 min.

Samples were separated using 1D-gel electrophoresis on pre-cast 8-16% gradient gels purchased from Bio-Rad (Bio-Rad Laboratories, Hemel Hempstead, UK). A sample containing 30-50 micrograms of the protein mixtures obtained from a detergent extract were applied to the stacking gel wells using a micro-pipette. A well containing molecular weight markers (10, 15, 25, 37, 50, 75, 100, 150 and 250 kDa) was included for calibration by interpolation of the separating gel after imaging. Separation of the proteins was performed by applying a current of 30mA to the gel for approximately 5 hours or until the bromophenol blue marker dye had reached the bottom of the gel.

After electrophoresis the gel plates were prised open, the gel placed in a tray of fixer (10% acetic acid, 40% ethanol, 50% water) and shaken overnight. The gel was then primed for 30 minutes by shaking in a primer solution (7.5% acetic acid, 0.05% SDS in Milli-Q water) followed by incubation with a fluorescent dye (0.06% OGS dye in 7.5% acetic acid) with shaking for 3 hrs. A preferred fluorescent dye is disclosed in U.S. 6,335,446. Sypro Red (Molecular Probes, Inc., Eugene, Oregon) is a suitable alternative dye for this purpose.

A digital image of the stained gel was obtained by scanning on a Storm Scanner (Molecular Dynamics Inc, USA) in the blue fluorescence mode. The captured image was used to determine the area of the gel to excise for in-gel proteolysis.

### 1e - Recovery and analysis of selected proteins

A vertical lane of the gel corresponding to 77kDa was excised using either a stainless steel scalpel blade or a PEEK gel cutter (OGS) that cuts sequentially down the length of the gel lane with no attempt at collecting specific protein bands.

Proteins were processed using in-gel digestion with trypsin (Modified trypsin, Promega, Wisconsin, USA) to generate tryptic digest peptides. Recovered samples were divided into two. Prior to MALDI analysis samples were desalted and concentrated using C18 Zip Tips^{™} (Millipore, Bedford, MA). Samples for tandem mass spectrometry were purified using a nano LC system (LC Packings, Amsterdam, The Netherlands) incorporating C18 SPE material. Recovered peptide pools were analysed by MALDI-TOF-mass spectrometry (Voyager STR, Applied Biosystems, Framingham, MA) using a 337 nm wavelength laser for desorption and the reflectron mode of analysis. Pools were also analyzed by nano-LC tandem mass spectrometry (LC/MS/MS) using a Micromass Quadrupole Time-of-Flight (Q-TOF) mass spectrometer (Micromass, Altrincham, UK). For partial amino acid sequencing and identification of liver and lung cancer cell membrane proteins uninterpreted tandem mass spectra of tryptic peptides were searched against a database of public domain proteins constructed of protein entries in the non-redundant database held by the National Centre for Biotechnology Information (NCBI) which is accessible at http://www.ncbi.nhD.nih.gov/ using the SEQUEST search program (Eng *et al.*, 1994, J. Am. Soc. Mass Spectrom 5:976-989), version v.C.1. Criteria for database identification included: the cleavage specificity of trypsin and the detection of a suite of a, b and y ions in peptides returned from the database. Following identification of proteins through spectral-spectral correlation using the SEQUEST program, masses detected in MALDI-TOF mass spectra were assigned to tryptic digest peptides within the proteins identified. In cases where no amino acid sequences could be identified through searching with uninterpreted MS/MS spectra of tryptic digest peptides using the SEQUEST program, tandem mass spectra of the peptides were interpreted manually, using methods known in the art. (In the case of interpretation of low-energy fragmentation mass spectra of peptide ions see Gaskell *et al.,* 1992, Rapid Commun. Mass Spectrom. 6:658-662). The method described in WO 02/21139 was also used to interpret mass spectra.

Two tandem spectra (shown in bold and underlined in Figure 1) and 1 mass match (shown in bold in Figure 1) were found to match the GenBank accession numbers (AF095448 and AAC98506, and SwissProt O95357.

### Example 2: Normal tissue distribution and disease tissue upregulation of RAIG1 using quantitative RT-PCR (Taqman) analysis

Real time RT-PCR was used to quantitatively measure RAIG1 expression in a range of tumour tissues and matched controls. Ethical approval for the normal and breast tumour tissue samples was obtained at surgery (University of Oxford, UK). The colon, lung, ovary serous cystadenocarcinoma, ovary adenocarcinoma samples, metastic osteosarcoma and pancreatic tumour samples were obtained from Ardais Corp., Peterborough Tissue Bank, Human Research Tissue Bank, Peterborough District Hospital, UK and Clinomics Biosciences Inc., MD. The primers used for PCR were as follows:
Sense, 5'-ctcgtgaagaagagctatggtc-3', (SEQ ID NO: 3)
Antisense, 5'- cactcttcaggacagagttagc - 3' (SEQ ID NO: 4)

Reactions containing 5ng cDNA, SYBR green sequence detection reagents (PE Biosystems) and sense and antisense primers were assayed on an ABI7700 sequence detection system (PE Biosystems). The PCR conditions were 1 cycle at 50°C for 2 min, 1 cycle at 95°C for 10 min, and 40 cycles of 95°C for 15s, 65°C for lmin. The accumulation of PCR product was measured in real time as the increase in SYBR green fluorescence, and the data were analysed using the Sequence Detector program v1.6.3 (PE Biosystems). Standard curves relating initial template copy number to fluorescence and amplification cycle were generated using the amplified PCR product as a template, and were used to calculate RAIG1 copy number in each sample.

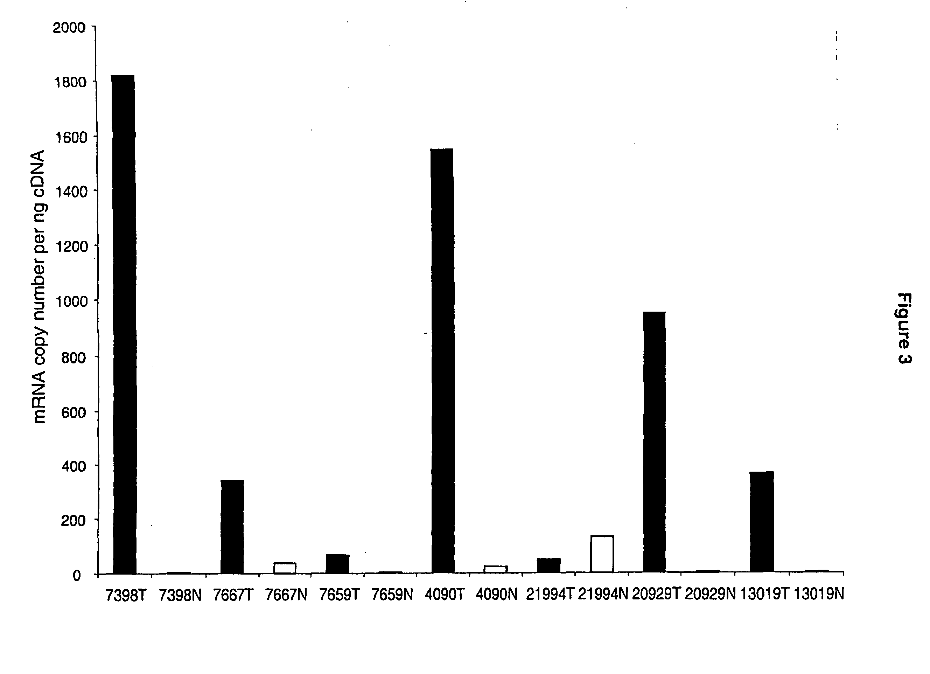

Relatively low expression levels of RAIG1 were seen in normal tissues (Figures 3-8; note that the scales are different between Figures).

In contrast, levels of RAIG1 expression were increased in breast tumour samples relative to their matched controls with 5/7 tumour samples showing greatly increased expression levels per ng cDNA (Figure 3).

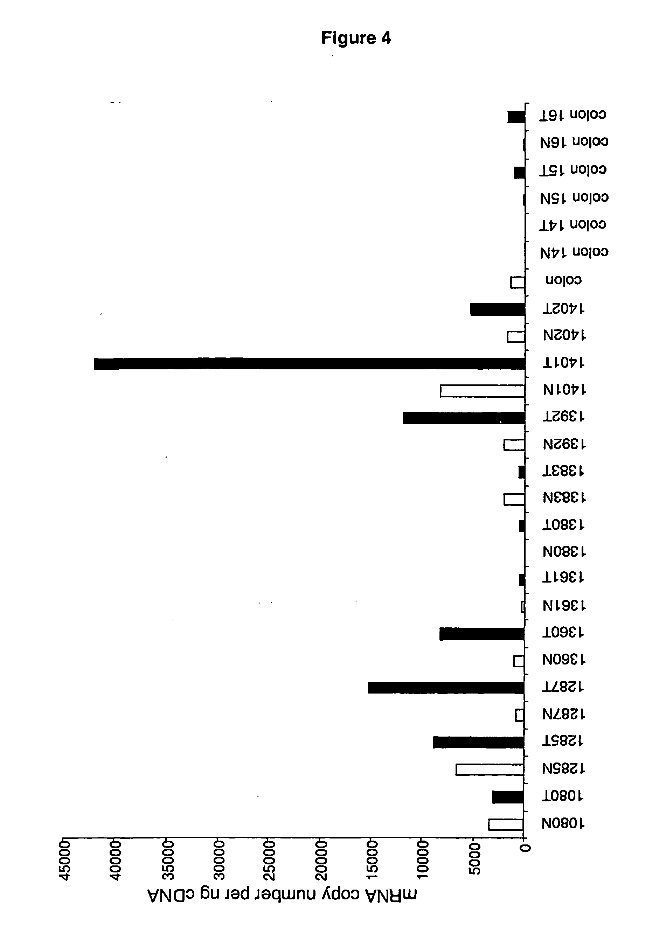

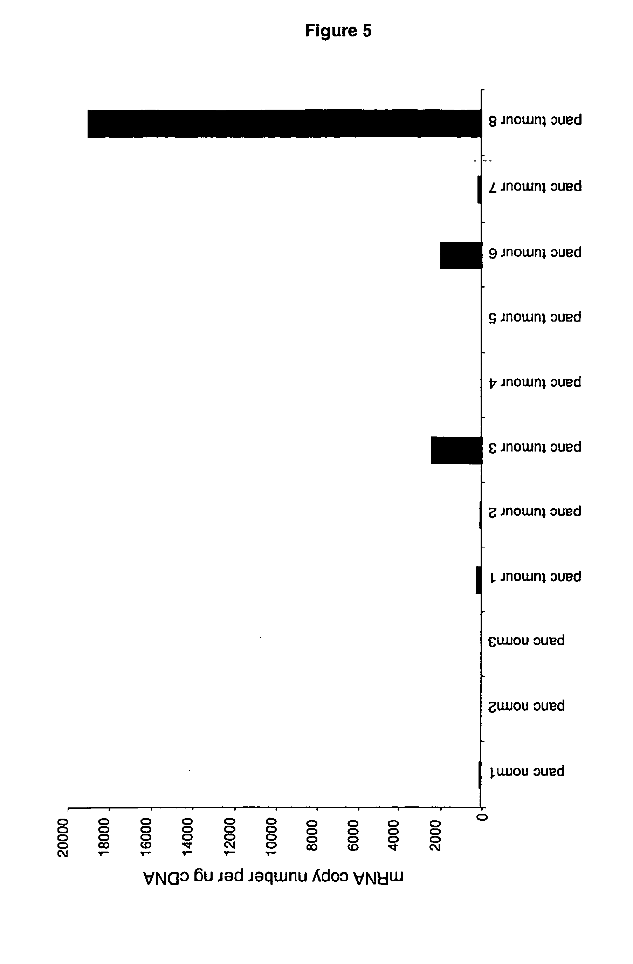

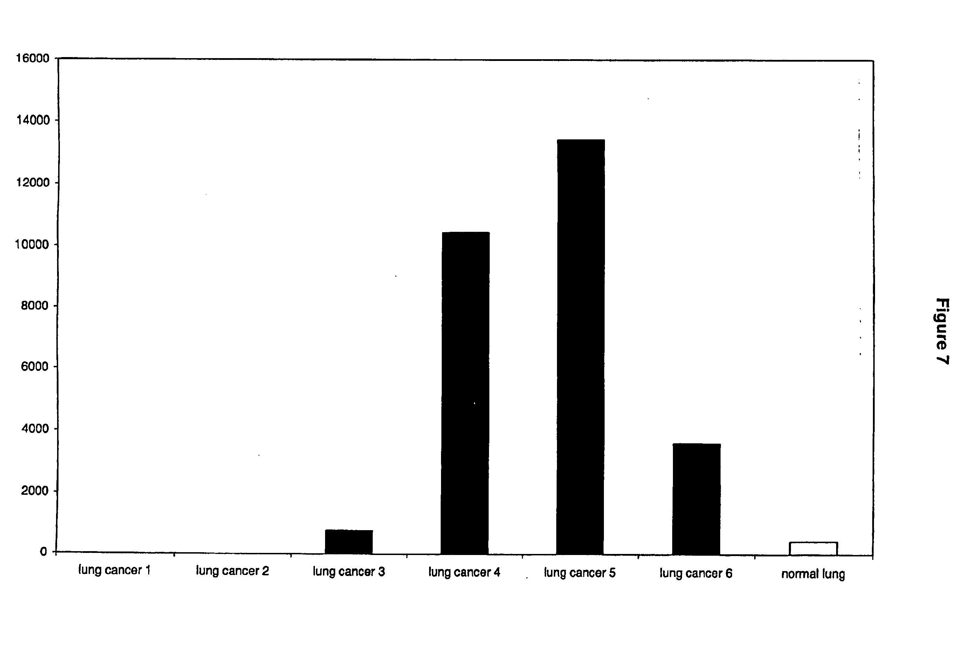

The expression of RAIG1 expression was examined in thirteen colon tumour samples and an increased expression was seen in ten samples relative to a control sample of normal colon with two showing little change and one tumour sample showing a small decrease (Figure 4). In pancreatic tumour samples, an increase in RAIG1 mRNA expression was seen in six out of eight tumour samples relative to control pancreatic tissue (Figure 5). In lung tumour samples, 4/6 samples showed an increased RAIG1 expression relative to a control lung tissue sample (Figure 7).

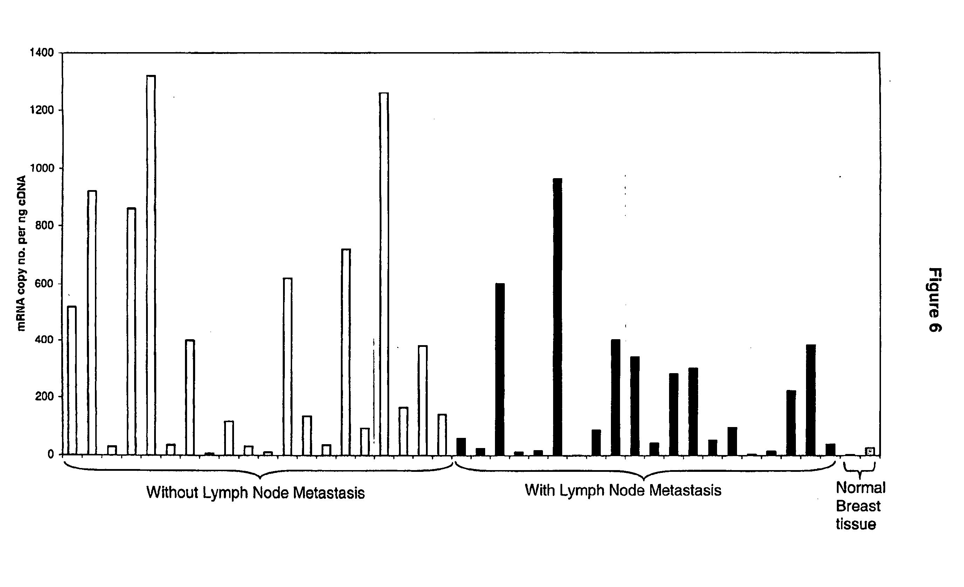

Additionally, the expression of RAIG1 was investigated in breast tissue samples from 20 patients with lymph node metastases and 20 patients without lymph node metastases. No significant difference between the two groups was observed, although a comparison with normal breast tissue samples indicates that RAIG1 is upregulated in a proportion of patients with and without lymph node metastases when compared to controls (Figure 6).

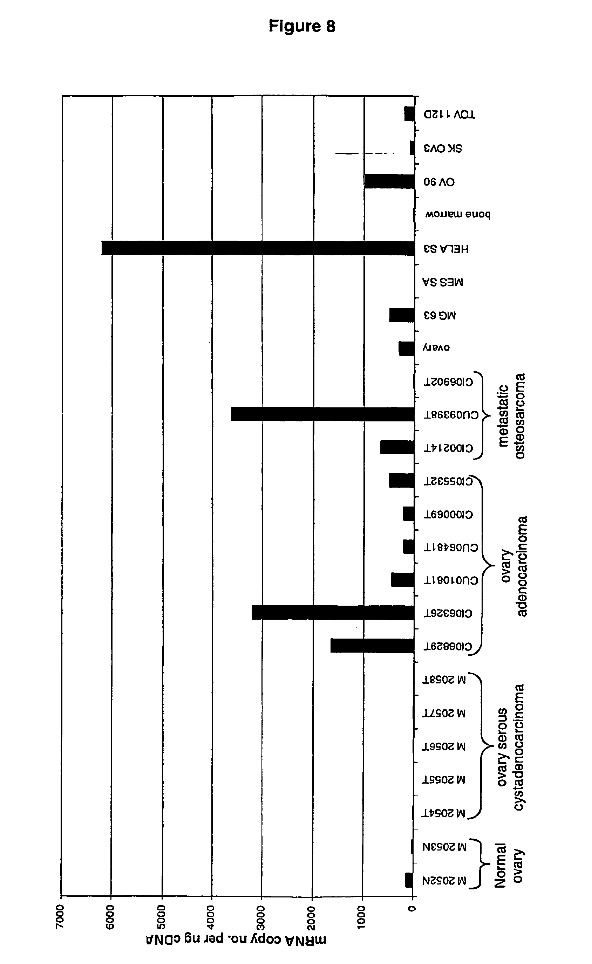

The expression of RAIG1 mRNA was examined in 5 ovary serous cystadenocarcinoma samples, 6 ovary adenocarcinoma samples, 3 metastatic osteosarcoma samples and in control tissue and cell lines. An increase in RAIG1 mRNA expression levels was seen in 2/6 of the ovary adenocarcinoma samples and 2/3 of the metastatic osteosarcoma samples but no increase was seen in the ovary serous cystadenocarcinoma samples (Figure 8).

This data demonstrates that RAIG1 is expressed in a number of tumourigenic cell lines, including breast, liver and pancreatic cell lines and that it is increased in a selection of carcinomas including breast cancer, colon cancer, ovary adenocarcinoma, osteosarcoma, lung cancer and pancreatic cancer indicating that RAIG1 is likely to be of utility as a carcinoma target. This finding could not have been predicted from previously reported findings on the expression of RAIG1 mRNA levels, RAIG1 was initially identified using differential display as the cDNA of a novel gene with high levels of mRNA expression in foetal and adult lung tissues (Cheng, Y. & Lotan, R., 1998, J. Biol. Chem. 273:35008-35015). Its expression has been reported to be induced by all-trans retinoic acid (ATRA). Three of five head and neck and four lung cancer cell lines which had low RAIG1 mRNA levels exhibited increased mRNA on treatment with ATRA (Cheng & Lotan, above), however, the expression levels of RAIG1 demonstrated by these authors was extremely variable (from undetectable to high levels of expression). Another group has reported that RAIG1 shows a restricted pattern of expression with the highest abundance in lung tissue (Brauner-0sbourne, H. & Krogsgaard-Larsen, P. 2000, Genomics, 65:121-128), in contrast, although we did find low levels of expression in control tissue samples we did not find a higher expression of RAIG1 mRNA in normal lung tissue, particularly when compared to the levels exhibited by the tumour samples. Therefore, theseresults clearly demonstrate an increased expression of RAIG1 mRNA in a variety of carcinoma types indicating its utility in the diagnosis, treatment and/or prophylaxis of carcinoma, e.g. breast cancer, pancreatic cancer, lung cancer, liver cancer, ovarian cancer, colon cancer and/or osteosarcoma, which would not have been obvious from the literature.

## Claims

1. A method of screening for breast cancer, colon cancer and/or osteosarcoma in a subject, and/or monitoring the effectiveness of an anti-breast cancer, anti-colon cancer and/or anti-osteosarcoma therapy, which comprises the step of detecting and/or quantifying in a biological sample obtained from said subject a RAIG1 polypeptide which comprises or consists of the amino acid sequence of SEQ ID NO:1, wherein said RAIG1 polypeptide is detected and/or quantified using an antibody that specifically binds to said RAIG1 polypeptide.

2. The method of claim 1, wherein the level of said polypeptide is compared to a previously determined reference range or control.

3. The method according to claim 1 or 2, wherein the antibody is immobilised on a solid phase.

4. The use of an antibody which specifically binds to a RAIG1 polypeptide as defined in claim in the manufacture of a medicament for the treatment of breast cancer, colon cancer and/or osteosarcoma, wherein the antibody is conjugated to a therapeutic or drug moiety.

5. The use according to claim 4, wherein the antibody is polyclonal, monoclonal, chimeric, humanised or bispecific, or an epitope-binding fragment of any one thereof.

6. The use according to claim 4 or 5, wherein the therapeutic or drug moiety is a radionuclide or a toxin.

7. A method of screening for breast cancer, colon cancer and/or osteosarcoma in a subject, and/or monitoring the effectiveness of an anti-breast cancer, anti-colon cancer and/or anti-osteosarcoma therapy, which comprises the step of detecting in a biological sample obtained from said subject, a nucleic acid which:
a) comprises or consists of the DNA sequence of SEQ ID NO:2, or its RNA equivalent;
b) has a sequence which is complementary to a sequence of a); or
c) has a sequence which codes for a polypeptide as defined in claim 1.

8. The method of claim 7, wherein the level of said nucleic acid is compared to a previously determined reference range or control.

## Patentansprüche

1. Verfahren zum Screening auf Brustkrebs, Darmkrebs und/oder Osteosarkom in einem Individuum und/oder zur Überwachung der Wirksamkeit einer Anti-Brustkrebs-, Anti-Darmkrebs- und/oder Anti-Osteosarkom-Therapie, wobei man in dem Verfahren in einer dem Individuum entnommenen biologischen Probe ein RAIG1-Polypeptid, das die Aminosäuresequenz der SEQ ID NO:1 umfaßt oder daraus besteht, nachweist und/oder quantifiziert, wobei das RAIG1-Polypeptid unter Verwendung eines an das RAIG1-Polypeptid spezifisch bindenden Antikörpers nachgewiesen und/oder quantifiziert wird.

2. Verfahren nach Anspruch 1, wobei der Spiegel des Polypeptids mit einem zuvor bestimmten Referenzbereich bzw. einer zuvor bestimmten Referenzkontrolle verglichen wird.

3. Verfahren nach Anspruch 1 oder 2, wobei der Antikörper auf einer Festphase immobilisiert ist.

4. Verwendung eines an ein RAIG1-Polypeptid spezifisch bindenden Antikörpers mit der in Anspruch 1 angegebenen Bedeutung bei der Herstellung eines Arzneimittels zur Behandlung von Brustkrebs, Darmkrebs und/oder Osteosarkom, wobei der Antikörper an eine therapeutische oder Arzneistoffgruppierung konjugiert ist.

5. Verwendung nach Anspruch 4, wobei es sich bei dem Antikörper um einen polyklonalen, monoklonalen, chimärischen, humanisierten oder bispezifischen Antikörper oder ein epitopbindendes Fragment eines dieser Antikörper handelt.

6. Verwendung nach Anspruch 4 oder 5, wobei es sich bei der therapeutischen oder Arzneistoffgruppierung um ein Radionuklid oder ein Toxin handelt.

7. Verfahren zum Screening auf Brustkrebs, Darmkrebs und/oder Osteosarkom in einem Individuum und/oder zur Überwachung der Wirksamkeit einer Anti-Brustkrebs-, Anti-Darmkrebs- und/oder Anti-Osteosarkom-Therapie, wobei man in dem Verfahren in einer dem Individuum entnommenen biologischen Probe eine Nukleinsäure nachweist, die:
a) die DNA-Sequenz der SEQ ID NO:2 oder deren RNA-Äquivalent umfaßt oder daraus besteht;
b) eine Sequenz aufweist, die zu einer Sequenz aus
a) komplementär ist; oder
c) eine Sequenz aufweist, die für ein Polypeptid mit der in Anspruch 1 angegebenen Bedeutung codiert.

8. Verfahren nach Anspruch 7, wobei der Spiegel der Nukleinsäure mit einem zuvor bestimmten Referenzbereich bzw. einer zuvor bestimmten Referenzkontrolle verglichen wird.

## Revendications

1. Procédé de dépistage du cancer du sein, du cancer du côlon et/ou de l'ostéosarcome chez un sujet, et/ou de surveillance de l'efficacité d'une thérapie anti-cancer du sein, anti-cancer du côlon et/ou anti-ostéosarcome, qui comprend l'étape de détection et/ou de quantification, dans un échantillon biologique obtenu à partir dudit sujet, d'un polypeptide RAIG1 qui comprend ou est constitué de la séquence d'acides aminés de SEQ ID NO:1, **caractérisé en ce que** ledit polypeptide RAIG1 est détecté et/ou quantifié en utilisant un anticorps qui se lie de manière spécifique audit polypeptide RAIG1.

2. Procédé selon la revendication 1, **caractérisé en ce que** le taux dudit polypeptide est comparé à une gamme de référence préalablement déterminée ou un témoin.

3. Procédé selon la revendication 1 ou 2, **caractérisé en ce que** l'anticorps est immobilisé sur une phase solide.

4. Utilisation d'un anticorps qui se lie de manière spécifique à un polypeptide RAIG1 tel que défini dans la revendication 1, pour la fabrication d'un médicament destiné au traitement du cancer du sein, du cancer du côlon et/ou de l'ostéosarcome, **caractérisée en ce que** l'anticorps est conjugué à un motif thérapeutique ou de médicament.

5. Utilisation selon la revendication 4, **caractérisée en ce que** l'anticorps est polyclonal, monoclonal, chimère, humanisé ou bispécifique, ou un fragment de l'un quelconque de ceux-ci se liant à un épitope.

6. Utilisation selon la revendication 4 ou 5, **caractérisée en ce que** le motif thérapeutique ou de médicament est un radionucléide ou une toxine.

7. Procédé de dépistage du cancer du sein, du cancer du côlon et/ou de l'ostéosarcome chez un sujet, et/ou de surveillance de l'efficacité d'une thérapie anti-cancer du sein, anti-cancer du côlon et/ou anti-ostéosarcome, qui comprend l'étape de détection, dans un échantillon biologique obtenu à partir dudit sujet, d'un acide nucléique qui :
a) comprend ou est constitué de la séquence d'ADN de SEQ ID NO:2, ou de son équivalent ARN ;
b) a une séquence qui est complémentaire à la séquence en a) ; ou
c) a une séquence qui code pour un polypeptide tel que défini dans la revendication 1.

8. Procédé selon la revendication 7, **caractérisé en ce que** le taux dudit acide nucléique est comparé à une gamme de référence préalablement déterminée ou un témoin.
